# EUROPEAN PATENT SPECIFICATION

(11) **EP 2 478 114 B1**
(45) Date of publication and mention of the grant of the patent: **17.12.2014**
(21) Application number: 10754329.0
(22) Date of filing: 16.09.2010
(51) Int. Cl.: C12Q 1/68

(54) **DIAGNOSTIC METHODS FOR LUNG CANCER**
VERFAHREN ZUR LUNG KREBSDIAGNOSTIK
MÉTHODES POUR LE DIAGNOSTIC DU CANCER DU POUMON

(30) Priority: 17.09.2009 US 243361 P
(43) Date of publication of application: 25.07.2012
(73) Proprietor: F.Hoffmann-La Roche AG, 4070 Basel (CH)
(72) Inventor: DE HAAS, Sanne Lysbet, CH-4051 Basel (CH); DELMAR, Paul, CH-4057 Basel (CH); SCHERER, Stefan, South San Francisco, California 94080 (US)
(74) Representative: Brodbeck, Michel
(86) International application number: PCT/EP2010/063584
(87) International publication number: WO 2011/033006

(56) References cited:
- DOWLATI A ET AL: "Cell adhesion molecules, vascular endothelial growth factor, and basic fibroblast growth factor in patients with non-small cell lung cancer treated with chemotherapy with or without bevacizumab - An Eastern Cooperative Oncology Group study" CLINICAL CANCER RESEARCH 20080301 US LNKD- DOI:10.1158/1078-0432.CCR-07-1154, vol. 14, no. 5, 1 March 2008 (2008-03-01), pages 1407-1412, XP002607505 ISSN: 1078-0432
- BHUVANESWARI R ET AL: "Hypericin-mediated photodynamic therapy in combination with Avastin (bevacizumab) improves tumor response by downregulating angiogenic proteins" PHOTOCHEMICAL AND PHOTOBIOLOGICAL SCIENCES, ROYAL SOCIETY OF CHEMISTRY, CAMBRIDGE, GB LNKD- DOI:10.1039/B705763F, vol. 6, no. 12, 1 December 2006 (2006-12-01), pages 1275-1283, XP008115107 ISSN: 1474-905X [retrieved on 2007-11-05]
- LI XIPING ET AL: "Thalidomide down-regulates the expression of VEGF and bFGF in cisplatin-resistant human lung carcinoma cells" ANTICANCER RESEARCH, INTERNATIONAL INSTITUTE OF ANTICANCER RESEARCH, GR, vol. 23, no. 3B, 1 May 2003 (2003-05-01), pages 2481-2487, XP009140123 ISSN: 0250-7005
- TARASENKO NATALY ET AL: "Histone deacetylase inhibitors: the anticancer, antimetastatic and antiangiogenic activities of AN-7 are superior to those of the clinically tested AN-9 (Pivanex)." CLINICAL & EXPERIMENTAL METASTASIS 2008 LNKD- PUBMED:18506586, vol. 25, no. 7, 2008, pages 703-716, XP002607506 ISSN: 0262-0898
- ROFSTAD E K ET AL: "Vascular endothelial growth factor, interleukin 8, platelet-derived endothelial cell growth factor, and basic fibroblast growth factor promote angiogenesis and metastasis in human melanoma xenografts" CANCER RESEARCH, AMERICAN ASSOCIATION FOR CANCER REREARCH, US, vol. 60, no. 17, 1 September 2000 (2000-09-01), pages 4932-4938, XP002359194 ISSN: 0008-5472
- HERBST R S ET AL: "Bevacizumab and erlotinib: A promising new approach to the treatment of advanced NSCLC" ONCOLOGIST 200811 US LNKD- DOI:10.1634/THEONCOLOGIST.2008-0108, vol. 13, no. 11, November 2008 (2008-11), pages 1166-1176, XP002607507
- MANEGOLD CHRISTIAN: "Bevacizumab for the treatment of advanced non-small-cell lung cancer." EXPERT REVIEW OF ANTICANCER THERAPY MAY 2008 LNKD- PUBMED:18471042, vol. 8, no. 5, May 2008 (2008-05), pages 689-699, XP009140141 ISSN: 1744-8328
- MANEGOLD CHRISTIAN: "New options for integrating antiangiogenic therapy and platinum-based first-line chemotherapy for advanced non-small-cell lung cancer." CLINICAL LUNG CANCER 2008 LNKD- PUBMED:19419923, vol. 9 Suppl 3, 2008, pages S100-S108, XP002607508 ISSN: 1938-0690
- RECK M ET AL: "Phase III trial of cisplatin plus gemcitabine with either placebo or bevacizumab as first-line therapy for nonsquamous non-small-cell lung cancer: AVAiL" JOURNAL OF CLINICAL ONCOLOGY 20090310 US LNKD- DOI:10.1200/JCO.2007.14.5466, vol. 27, no. 8, 10 March 2009 (2009-03-10) , pages 1227-1234, XP002607509 ISSN: 0732-183X

## Description

The present invention relates to methods and compositions useful for predicting clinical outcome and for monitoring cancer patients treated with anti-angiogenic therapy.

Cancer is one of the most deadly threats to human health. In the U.S. alone, cancer affects nearly 1.3 million new patients each year, and is the second leading cause of death after cardiovascular disease, accounting for approximately 1 in 4 deaths. Solid tumors are responsible for most of those deaths. Although there have been significant advances in the medical treatment of certain cancers, the overall 5-year survival rate for all cancers has improved only by about 10% in the past 20 years. Cancers, or malignant tumors, metastasize and grow rapidly in an uncontrolled manner, making timely detection and treatment extremely difficult.

Depending on the cancer type, patients typically have several treatment options available to them including chemotherapy, radiation and antibody-based drugs. Diagnostic methods useful for predicting clinical outcome from the different treatment regimens would greatly benefit clinical management of these patients. Several studies have explored the correlation of gene expression with the identification of specific cancer types, e.g., by mutation-specific assays, microarray analysis, qPCR, etc. Such methods may be useful for the identification and classification of cancer presented by a patient. However, much less is known about the predictive or prognostic value of gene expression with clinical outcome.

Thus, there is a need for objective, reproducible methods for predicting treatment outcome and thereby selecting the optimal treatment regimen for each patient.

Dowlati A et al., Clin Cancer Res (2008); 14(5):1407-12 relates to a correlative study of different markers, i.e. VEGF, bFGF, ICAM and E-selectin, in patients with non-small cell lung cancer treated with bevacizumab but does not show an association with bFGF levels and response to bevacizumab.

Herbst R S et al., Oncologist (2008); 13(11):1166-76 describes that correlative study was carried out in NSCLC patients enrolled in a randomized phase II/III trial of paclitaxel plus carboplatin with or without bevacizumab. Levels of VEGF. basic fibroblast growth factor, and soluble intercellular adhesion molecule-1 (ICAM) were examined. Only baseline ICAM levels appeared to be a prognostic factor, being strongly prognostic for survival and response to

### Chemotherapy.

The methods of the present invention can be utilized in a variety of settings, including, for example, in aiding in patient selection during the course of drug development, in selecting the optimal treatment course for a patient, prediction of likelihood of success when treating an individual patient with a particular treatment regimen, in assessing disease progression, in monitoring treatment efficacy, in determining prognosis for individual patients and in assessing predisposition of an individual to benefit from a particular therapy, e.g., an anti-cancer therapy and/or anti-angiogenic therapy.

The present invention is based, in part, on the discovery that expression levels of bFGF in patients suffering from cancer correlate with increased clinical benefit from anti-angiogenic therapy. Accordingly, one aspect the invention provides methods of identifying a cancer patient who may benefit from anti-angiogenic therapy, comprising determining expression level of bFGF in a sample obtained from the patient, wherein higher expression level of bFGF in the sample obtained from the patient as compared to a reference sample indicates that the patient may benefit from anti-angiogenic therapy.

Another aspect the invention provides methods of predicting responsiveness of a patient with a cancer to anti-angiogenic therapy comprising determining expression level of bFGF in a sample obtained from the patient, wherein higher expression level bFGF in the sample as compared to a reference sample indicates that the patient is likely to be responsive to the anti-angiogenic therapy.

In certain embodiments, the anti-angiogenic therapy comprises administering a VEGF antagonist or a fragment thereof In certain embodiments, the VEGF antagonist is an anti-VEGF antibody. In certain embodiments, the anti-VEGF antibody is a monoclonal antibody. In certain embodiments, the anti-VEGF antibody is bevacizumab. In certain embodiments, the anti-angiogenic therapy comprises administering 5 mg/kg, 7.5 mg/kg, 10 mg/kg or 15 mg/kg of bevacizumab. In certain embodiments, the anti-angiogenic therapy further comprises administering at least one chemotherapeutic agent to the patient. In certain embodiments, at least one of the chemotherapeutic agents is cisplatin or gemcitabine. In certain embodiments, the anti-angiogenic therapy further comprises administering at least two chemotherapeutic agents to the patient. In certain embodiments, at least two chemotherapeutic agents are cisplatin and gemcitabine.

In one embodiment, the higher expression level of bFGF in the sample obtained from the patient as compared to the reference sample indicates that the patient may benefit from anti-angiogenic therapy comprising administration of 7.5 mg/kg of bevacizumab. In another embodiment, the higher expression level bFGF in the sample as compared to the reference sample indicates that the patient is likely to be responsive to the anti-angiogenic therapy comprising administration of 7.5 mg/kg of bevacizumab.

In certain embodiments, the methods of the present disclosure further comprise administering an effective amount of anti-VEGF antibody to the patient. In certain embodiments, the anti-VEGF antibody is bevacizumab. In certain embodiments, the effective amount of bevacizumab that is administered to the patient is 7.5 mg/kg of bevacizumab. In certain embodiments, the methods of the present disclosure further comprise administering an effective amount of at least one chemotherapeutic agent to the patient. In certain embodiments, at least one of the chemotherapeutic agent is cisplatin or gemcitabine. In certain embodiments, the methods of the present disclosure further comprise administering an effective amount of at least two chemotherapeutic agents to the patient. In certain embodiments, at least two chemotherapeutic agents are cisplatin and gemcitabine.

The present disclosure provides methods of treating a patient with an anti-VEGF antibody, comprising (1) determining expression level of bFGF in a sample obtained from the patient and (2) administering an effective amount of anti-VEGF antibody to the patient if there is a higher expression level of bFGF in the sample as compared to a reference sample.

In certain embodiments, the expression level of bFGF being measured is protein expression level. In certain embodiments, the protein expression level is measured using an immunological assay. In certain embodiments, the immunological assay is ELISA. In certain embodiments, the expression level of bFGF being measured is mRNA expression level.

In certain embodiments, the protein expression level of bFGF in the sample is greater than about 2 pg/ml. In certain embodiments, the protein expression level of bFGF in the sample is greater than about 4 pg/ml. In certain embodiments, the protein expression level of bFGF in the sample is greater than about 6 pg/ml. In certain embodiments, the protein expression level of bFGF in the sample is greater than about 6.9 pg/ml. In certain embodiments, the protein expression level of bFGF in the reference sample is equal to or less than about 2 pg/ml. In certain embodiments, the protein expression level of bFGF in the reference sample is equal to or less than about 4 pg/ml. In certain embodiments, the protein expression level of bFGF in the reference sample is equal to or less than about 6 pg/ml. In certain embodiments, the protein expression level of bFGF in the reference sample is equal to or less than about 6.9 pg/ml.

The present disclosure further provides methods of treating a cancer patient comprising determining protein expression level of bFGF in a sample obtained from the patient and administering an effective amount of an anti-VEGF antibody to the patient if the bFGF protein expression level is greater than about 2 pg/ml. In certain embodiments, the bFGF protein expression level in the sample is greater than about 4 pg/ml. In certain embodiments, the bFGF protein expression level in the sample is greater than about 6 pg/ml. In certain embodiments, the bFGF protein expression level in the sample is greater than about 6.9 pg/ml.

In certain embodiments, the anti-VEGF antibody administered to the patient is a monoclonal antibody. In certain embodiments, the anti-VEGF antibody administered to the patient is a humanized antibody. In certain embodiments, the anti-VEGF antibody is bevacizumab or fragment thereof In certain embodiments, the patient is administered 5 mg/kg, 7.5 mg/kg, 10 mg/kg or 15 mg/kg of bevacizumab. In certain embodiments, the effective amount of bevacizumab that is administered to the patient is 7.5 mg/kg of bevacizumab.

The methods of the present disclosure further comprise administering to the patient an effective amount of at least one chemotherapeutic agent. In certain embodiments, the chemotherapeutic agent is cisplatin. In certain embodiments, the chemotherapeutic agent is gemcitabine. In certain embodiments, the chemotherapeutic agent is carboplatin. In certain embodiments, the chemotherapeutic agent is paclitaxel. In certain embodiments, the chemotherapeutic agent is pemetrexed. In certain embodiments, the methods of present disclosure further comprise administering to the patient effective amounts of cisplatin and gemcitabine. In certain embodiments, the methods of present disclosure further comprise administering to the patient effective amounts of carboplatin and paclitaxel.

In certain embodiments, the cancer is breast cancer, colon cancer, ovarian cancer, renal cancer or glioblastoma. The cancer is lung cancer, in certain embodiments, the cancer is previously untreated lung cancer. In certain embodiments, the lung cancer is non-small cell lung cancer. In certain embodiments, the lung cancer is previously untreated non-small cell lung cancer.

In certain embodiments, the sample obtained from the patient is a member selected from the group consisting of: tissue, blood-derived cells, plasma, serum, and combinations thereof. The sample is blood sample. In certain embodiments, the sample from the patient is obtained before or at commencement of the anti-angiogenic therapy.

In certain embodiments, the expression level of bFGF being measured is the bFGF protein level in blood. In certain embodiments, bFGF protein is the mature form of bFGF protein.

The disclosure provides sets of compounds for detecting expression level of bFGF in a sample obtained from a cancer patient. The sets comprise one or more compounds capable of detecting the expression level of bFGF, wherein higher expression level of bFGF, determined using the set of one or more compounds, in a sample obtained from a patient with cancer as compared to a reference sample indicates that the patient may benefit from anti-angiogenic therapy. In certain embodiments, the compounds are proteins. In certain embodiments, the proteins are antibodies. In certain embodiment, at least one of the antibodies is capable of binding to bFGF.

The disclosure provides kits for determining whether a patient may benefit from treatment with an anti-angiogenic therapy. The kits comprise an array comprising polynucleotides capable of specifically hybridizing to bFGF, wherein the kit further comprises instructions for using said array to predict responsiveness of a patient with cancer to anti-angiogenic therapy, wherein higher expression level of bFGF as compared to a reference sample indicates that the patient may benefit from anti-angiogenic therapy.

Any embodiment described herein or any combination thereof applies to any and all methods of the invention described herein.

The following drawings are provided:
**Figure 1** shows AVAiL trial design.
**Figure 2** is Kaplan Meier curve showing the probability of progression free survival by treatment groups in lung cancer patients with high protein expression level of bFGF.
**Figure 3** is Kaplan Meier curve showing the probability of progression free survival by treatment groups in lung cancer patients with low protein expression level of bFGF.
**Figure 4** is Kaplan Meier curve showing the probability of overall survival by treatment groups in lung cancer patients with high protein expression level of bFGF.
**Figure 5** is Kaplan Meier curve showing the probability of overall survival by treatment groups in lung cancer patients with low protein expression level of bFGF.

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3rd. edition (2001) Cold Spring Harbor Laboratory Press, Cold Spring Harbor, N.Y. CURRENT PROTOCOLS IN MOLECULAR BIOLOGY (F. M. Ausubel, et al. eds., (2003)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. (1987)); Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Vulture (R. I. Freshney), ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-8) J. Wiley and Sons; Handbook of Experimental Immunology (D. M. Weir and C. C. Blackwell, eds.); Gene Transfer Vectors for Mammalian Cells (J. M. Miller and M. P. Calos, eds., 1987); PCR: The Polymerase Chain Reaction, (Mullis et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V. T. DeVita et al., eds., J.B. Lippincott Company, 1993).

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

For purposes of interpreting this specification, the following definitions will apply and whenever appropriate, terms used in the singular will also include the plural and vice versa.

The term "sample," or "test sample" as used herein, refers to a composition that is obtained or derived from a subject of interest that contains a cellular and/or other molecular entity that is to be characterized and/or identified, for example based on physical, biochemical, chemical and/or physiological characteristics. In one embodiment, the definition encompasses blood and other liquid samples of biological origin and tissue samples such as a biopsy specimen or tissue cultures or cells derived therefrom. The source of the tissue sample may be solid tissue as from a fresh, frozen and/or preserved organ or tissue sample or biopsy or aspirate; blood or any blood constituents; bodily fluids; and cells from any time in gestation or development of the subject or plasma.

In another embodiment, the definition includes biological samples that have been manipulated in any way after their procurement, such as by treatment with reagents, solubilization, or enrichment for certain components, such as proteins or polynucleotides, or embedding in a semi-solid or solid matrix for sectioning purposes. For the purposes herein a "section" of a tissue sample is meant a single part or piece of a tissue sample, *e.g.* a thin slice of tissue or cells cut from a tissue sample.

Samples include, but not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, urine, cerebro-spinal fluid, saliva, sputum, tears, perspiration, mucus, tumor lysates, and tissue culture medium, as well as tissue extracts such as homogenized tissue, tumor tissue, and cellular extracts.

In one embodiment, the sample is a clinical sample. In another embodiment, the sample is used in a diagnostic assay. The sample is a blood sample. In certain embodiments, the sample is a peripheral blood sample. In certain embodiments, the sample is a serum sample.

In certain embodiments, the sample is obtained from a primary or metastatic tumor. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood.

In one embodiment, a test sample is obtained from a subject or patient prior to anti-angiogenic therapy. In another embodiment, a test sample is obtained from a subject or patient prior to VEGF antagonist therapy. In yet another embodiment, a test sample is obtained from a subject or patient prior to anti-VEGF antibody therapy. In yet another embodiment, a test sample is obtained from a subject or patient prior to administering anti-VEGF antibody bevacizumab. In certain embodiment, a test sample is obtained during or after anti-angiogenic, VEGF antagonist or anti-VEGF antibody therapy. In certain embodiments, a test sample is obtained after cancer has metastasized.

A "reference sample," as used herein, refers to any sample, standard, or level that is used for comparison purposes. A reference sample includes all types of biological samples as defined above under the term "sample." In certain embodiments, a reference sample is a blood sample. In certain embodiments, a reference sample is a peripheral blood sample. In certain embodiments, a reference sample is a serum sample. In certain embodiments, a reference sample is a plasma sample. A reference sample can be either a selected sample or a pooled sample.

In one embodiment, a reference sample is obtained from a healthy and/or non-diseased part of the body of the same subject or patient. In another embodiment, a reference sample is obtained from an untreated tissue and/or cell of the body of the same subject or patient.

In certain embodiments, a reference sample is obtained from one or more individuals with cancer who is not the subject or patient. In one embodiment, a reference sample is obtained from a healthy and/or non-diseased part of the body of an individual who is not the subject or patient. In another embodiment, a reference sample is obtained from an untreated tissue and/or cell part of the body of an individual who is not the subject or patient.

In certain embodiments, a reference sample is representative of a combined multiple samples from one or more healthy individuals who are not the subject or patient. In certain embodiments, a reference sample is representative of a combined multiple samples from one or more individuals with cancer who are not the subject or patient. In certain embodiments, a reference sample is pooled protein and/or RNA samples from one or more individuals who are not the subject or patient.

In certain embodiments, a reference sample is a single sample or combined multiple samples from the same subject or patient that are obtained at one or more different time points than when the test sample is obtained. For example, a reference sample is obtained at an earlier time point from the same subject or patient than when the test sample is obtained. Such reference sample may be useful if the reference sample is obtained during initial diagnosis of cancer and the test sample is later obtained when the cancer becomes metastatic. In certain embodiments, a reference sample is obtained at a later time point from the same subject or patient than when the test sample is obtained.

An "individual," "subject," or "patient" is a vertebrate. In certain embodiments, the vertebrate is a mammal. Mammals include, but are not limited to, farm animals (such as cows), sport animals, pets (such as cats, dogs, and horses), primates, mice and rats. In certain embodiments, a mammal is a human.

"Detection" includes any means of detecting, including direct and indirect detection.

The word "label" when used herein refers to a compound or composition which is conjugated or fused directly or indirectly to a reagent such as a nucleic acid probe or an antibody and facilitates detection of the reagent to which it is conjugated or fused. The label may itself be detectable (*e.g*., radioisotope labels or fluorescent labels) or, in the case of an enzymatic label, may catalyze chemical alteration of a substrate compound or composition which is detectable.

A "target sequence," "target nucleic acid" or "target protein," as used herein, is a polynucleotide or protein of interest, the detection of which is desired. Generally, a "template," as used herein, is a polynucleotide that contains the target nucleotide sequence. In some instances, the terms "target sequence," "template DNA," "template polynucleotide," "target nucleic acid," "target polynucleotide," and variations thereof, are used interchangeably.

The term "biomarker" as used herein refers generally to a molecule, including a gene, protein, carbohydrate structure, or glycolipid, the expression of which in or on a mammalian tissue or cell can be detected by standard methods (or methods disclosed herein) and is predictive, diagnostic and/or prognostic for a subject's sensitivity to treatment regimes based on inhibition of angiogenesis e.g. an anti-angiogenesis agent such as a VEGF-specific inhibitor. In certain embodiments, the expression of such a biomarker is determined to be higher than that observed for a reference sample. In certain embodiments, the biomarker is bFGF. Expression of biomarkers can be determined, for example, using a high-throughput multiplexed immunoassay such as those commercially available from Rules Based Medicine, Inc. or Meso Scale Discovery. Expression of the biomarkers may also be determined using, e.g., PCR or FACS assay, an immunohistochemical assay or a gene chip-based assay. Additional methods for measuring expression of the biomarkers is described herein under Methods of the Invention.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of gene expression analysis or protocol, one may use the results of the gene expression analysis or protocol to determine whether a specific therapeutic regimen should be performed.

Expression level/amount of a gene, gene product, e.g., biomarker, can be determined based on any suitable criterion known in the art, including but not limited to mRNA, cDNA, proteins, protein fragments and/or gene copy. Expression levels/amounts can be determined qualitatively and/or quantitatively. In certain embodiments, the samples are normalized for both differences in the amount of RNA or protein assayed and variability in the quality of the RNA or protein samples used. Such normalization may be accomplished by measuring and incorporating the expression of certain normalizing genes, including well known housekeeping genes, such as GAPDH. Alternatively, normalization can be based on the mean or median signal of all of the assayed genes or a large subset thereof (global normalization approach). On a gene-by-gene basis, measured normalized amount of a patient tumor mRNA or protein is compared to the amount found in a reference set. Normalized expression levels for each mRNA or protein per tested tumor per patient can be expressed as a percentage of the expression level measured in the reference set. The expression level measured in a particular patient sample to be analyzed will fall at some percentile within this range, which can be determined by methods well known in the art.

The term "array" or "microarray", as used herein refers to an ordered arrangement of hybridizable array elements, preferably polynucleotide probes (e.g., oligonucleotides), on a substrate. The substrate can be a solid substrate, such as a glass slide, or a semi-solid substrate, such as nitrocellulose membrane. The nucleotide sequences can be DNA, RNA, or any permutations thereof.

"Amplification," as used herein, generally refers to the process of producing multiple copies of a desired sequence. "Multiple copies" mean at least 2 copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc. ), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-2'-O- allyl, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR₂ ("amidate"), P(O)R, P(O)OR', CO or CH₂ ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

A "primer" is generally a short single stranded polynucleotide, generally with a free 3'-OH group, that binds to a target potentially present in a sample of interest by hybridizing with a target sequence, and thereafter promotes polymerization of a polynucleotide complementary to the target.

A "native sequence" polypeptide comprises a polypeptide having the same amino acid sequence as a polypeptide derived from nature. Thus, a native sequence polypeptide can have the amino acid sequence of naturally occurring polypeptide from any mammal. Such native sequence polypeptide can be isolated from nature or can be produced by recombinant or synthetic means. The term "native sequence" polypeptide specifically encompasses naturally occurring truncated or secreted forms of the polypeptide (e.g., an extracellular domain sequence), naturally occurring variant forms (e.g., alternatively spliced forms) and naturally occurring allelic variants of the polypeptide.

An "isolated" polypeptide or "isolated" antibody is one that has been identified and separated and/or recovered from a component of its natural environment. Contaminant components of its natural environment are materials that would interfere with diagnostic or therapeutic uses for the polypeptide, and may include enzymes, hormones, and other proteinaceous or nonproteinaceous solutes. In certain embodiments, the polypeptide will be purified (1) to greater than 95% by weight of polypeptide as determined by the Lowry method, or more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue, or silver stain. Isolated polypeptide includes the polypeptide in situ within recombinant cells since at least one component of the polypeptide's natural environment will not be present. Ordinarily, however, isolated polypeptide will be prepared by at least one purification step.

A "polypeptide chain" is a polypeptide wherein each of the domains thereof is joined to other domain(s) by peptide bond(s), as opposed to non-covalent interactions or disulfide bonds.

A polypeptide "variant" means a biologically active polypeptide having at least about 80% amino acid sequence identity with the corresponding native sequence polypeptide. Such variants include, for instance, polypeptides wherein one or more amino acid (naturally occurring amino acid and/or a non-naturally occurring amino acid) residues are added, or deleted, at the N- and/or C-terminus of the polypeptide. Ordinarily, a variant will have at least about 80% amino acid sequence identity, or at least about 90% amino acid sequence identity, or at least about 95% or more amino acid sequence identity with the native sequence polypeptide. Variants also include polypeptide fragments (e.g., subsequences, truncations, etc.), typically biologically active, of the native sequence.

The term "protein variant" as used herein refers to a variant as described above and/or a protein which includes one or more amino acid mutations in the native protein sequence. Optionally, the one or more amino acid mutations include amino acid substitution(s). Protein and variants thereof for use in the invention can be prepared by a variety of methods well known in the art. Amino acid sequence variants of a protein can be prepared by mutations in the protein DNA. Such variants include, for example, deletions from, insertions into or substitutions of residues within the amino acid sequence of protein. Any combination of deletion, insertion, and substitution may be made to arrive at the final construct having the desired activity. The mutations that will be made in the DNA encoding the variant must not place the sequence out of reading frame and preferably will not create complementary regions that could produce secondary mRNA structure. *See e.g.,* EP 75,444A.

The term "antibody" is used in the broadest sense and specifically covers monoclonal antibodies (including full length or intact monoclonal antibodies), polyclonal antibodies, multivalent antibodies, multispecific antibodies (e.g., bispecific antibodies) formed from at least two intact antibodies, and antibody fragments (see below) so long as they exhibit the desired biological activity.

Unless indicated otherwise, the expression "multivalent antibody" is used throughout this specification to denote an antibody comprising three or more antigen binding sites. The multivalent antibody is typically engineered to have the three or more antigen binding sites and is generally not a native sequence IgM or IgA antibody.

"Antibody fragments" comprise only a portion of an intact antibody, generally including an antigen binding site of the intact antibody and thus retaining the ability to bind antigen. Examples of antibody fragments encompassed by the present definition include: (i) the Fab fragment, having V_{L}, C_{L}, V_{H} and C_{H}1 domains; (ii) the Fab' fragment, which is a Fab fragment having one or more cysteine residues at the C-terminus of the C_{H}1 domain; (iii) the Fd fragment having V_{H} and C_{H}1 domains; (iv) the Fd' fragment having V_{H} and C_{H}1 domains and one or more cysteine residues at the C-terminus of the C_{H}1 domain; (v) the Fv fragment having the V_{L} and V_{H} domains of a single arm of an antibody; (vi) the dAb fragment (Ward et al., Nature 341, 544-546 (1989)) which consists of a VH domain; (vii) isolated CDR regions; (viii) F(ab')2 fragments, a bivalent fragment including two Fab' fragments linked by a disulphide bridge at the hinge region; (ix) single chain antibody molecules (e.g. single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (V_{H}) connected to a light chain variable domain (V_{L}) in the same polypeptide chain (see, e.g., EP 404,097; WO 93/11161; and Hollinger et al., Proc. Natl. Acad. Sci. USA, 90:6444-6448 (1993)); (xi) "linear antibodies" comprising a pair of tandem Fd segments (V_{H}-C_{H}1-V_{H}-C_{H}1) which, together with complementary light chain polypeptides, form a pair of antigen binding regions (Zapata et al. Protein Eng. 8(10):1057 1062 (1995); and US Patent No. 5,641,870).

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. Monoclonal antibodies are highly specific, being directed against a single antigen. In certain embodiments, a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations that typically include different antibodies directed against different determinants (epitopes), each monoclonal antibody is directed against a single determinant on the antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler and Milstein, Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage-display technologies *(see, e.g.,* Clackson et al., Nature, 352: 624-628 (1991); Marks et al., J. Mol. Biol. 222: 581-597 (1991); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J: Immunol. Methods 284(1-2): 119-132(2004), and technologies for producing human or human-like antibodies in animals that have parts or all of the human immunoglobulin loci or genes encoding human immunoglobulin sequences (*see, e.g.,* WO 1998/24893; WO 1996/34096; WO 1996/33735; WO 1991/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also, *e.g.,* Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409. *See also* van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.,* immunized xenomice (see, *e.g*., U.S. Pat. Nos. 6,075,181 and 6,150,584 regarding XENOMOUSE™ technology). See also, for example, Li et al., Proc. Natl. Acad. Sci. USA, 103:3557-3562 (2006) regarding human antibodies generated via a human B-cell hybridoma technology.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. PNAS (USA) 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized *in vitro). See, e.g.,* Cole et al., Monoclonal Antibodies and Cancer Therapy, Alan R. Liss, p. 77 (1985); Boerner et al., J. Immunol., 147 (1):86-95 (1991); and US Pat No. 5,750,373.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a beta-sheet configuration, connected by three hypervariable regions, which form loops connecting, and in some cases forming part of, the beta-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody-dependent cellular toxicity.

The term "hypervariable region," "HVR," or "HV," when used herein refers to the amino acid residues of an antibody which are responsible for antigen-binding. For example, the term hypervariable region refers to the regions of an antibody variable domain which are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, e.g., Xu et al., Immunity 13:37-45 (2000); Johnson and Wu, in Methods in Molecular Biology 248:1-25 (Lo, ed., Human Press, Totowa, NJ, 2003). Indeed, naturally occurring camelid antibodies consisting of a heavy chain only are functional and stable in the absence of light chain. See, e.g., Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact | |
|---|---|---|---|---|---|
| L1 | L24-L34 | L24-L34 | L26-L32 | L30-L36 | |
| L2 | L50-L56 | L50-L56 | L50-L52 | L46-L55 | |
| L3 | L89-L97 | L89-L97 | L91-L96 | L89-L96 | |
| H1 | H31-H35B | H26-H35B | H26-H32 | H30-H35B | (Kabat Numbering) |
| H1 | H31-H35 | H26-H35 | H26-H32 | H30-H35 | (Chothia Numbering) |
| H2 | H50-H65 | H50-H58 | H53-H55 | H47-H58 | |
| H3 | H95-H102 | H95-H102 | H96-H101 | H93-H101 | |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat *et al., supra,* for each of these definitions.

"Framework Region" or "FR" residues are those variable domain residues other than the hypervariable region residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., *supra.* Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

Throughout the present specification, the Kabat numbering system is generally used when referring to a residue in the variable domain (approximately, residues 1-107 of the light chain and residues 1-113 of the heavy chain) (e.g., Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., Sequences of Proteins of Immunological Interest, 5th Ed. Public Health Service, National Institutes of Health, Bethesda, MD (1991)). Unless stated otherwise herein, references to residues numbers in the variable domain of antibodies means residue numbering by the Kabat numbering system. Unless stated otherwise herein, references to residue numbers in the constant domain of antibodies means residue numbering by the EU numbering system (for details see e.g. US Patent Application Publication No. 2998/0181888 which claims priority of United States Provisional Application No. 60/640,323).

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG₁ (including non-A and A allotypes), IgG₂, IgG₃, IgG₄, IgA₁, and IgA₂. The heavy chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (W.B. Saunders, Co., 2000). An antibody may be part of a larger fusion molecule, formed by covalent or non-covalent association of the antibody with one or more other proteins or peptides.

The "light chains" of antibodies (immunoglobulins) from any vertebrate species can be assigned to one of two clearly distinct types, called kappa (κ) and lambda (λ), based on the amino acid sequences of their constant domains.

The term "Fc region" is used to define the C-terminal region of an immunoglobulin heavy chain which may be generated by papain digestion of an intact antibody. The Fc region may be a native sequence Fc region or a variant Fc region. Although the boundaries of the Fc region of an immunoglobulin heavy chain might vary, the human IgG heavy chain Fc region is usually defined to stretch from an amino acid residue at about position Cys226, or from about position Pro230, to the carboxyl-terminus of the Fc region. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. The Fc region of an immunoglobulin generally comprises two constant domains, a C_{H}2 domain and a C_{H}3 domain, and optionally comprises a C_{H}4 domain.

Unless indicated otherwise herein, the numbering of the residues in an immunoglobulin heavy chain is that of the EU index as in Kabat et al., supra. The "EU index as in Kabat" refers to the residue numbering of the human IgG1 EU antibody.

By "Fc region chain" herein is meant one of the two polypeptide chains of an Fc region.

The "C_{H}2 domain" of a human IgG Fc region (also referred to as "Cg2" domain) usually extends from an amino acid residue at about position 231 to an amino acid residue at about position 340. The C_{H}2 domain is unique in that it is not closely paired with another domain. Rather, two N-linked branched carbohydrate chains are interposed between the two C_{H}2 domains of an intact native IgG molecule. It has been speculated that the carbohydrate may provide a substitute for the domain-domain pairing and help stabilize the C_{H}2 domain. Burton, *Molec. Immunol.*22:161-206 (1985). The C_{H}2 domain herein may be a native sequence C_{H}2 domain or variant C_{H}2 domain.

The "C_{H}3 domain" comprises the stretch of residues C-terminal to a C_{H}2 domain in an Fc region (i.e. from an amino acid residue at about position 341 to an amino acid residue at about position 447 of an IgG). The C_{H}3 region herein may be a native sequence C_{H}3 domain or a variant C_{H}3 domain (e.g. a C_{H}3 domain with an introduced "protroberance" in one chain thereof and a corresponding introduced "cavity" in the other chain thereof; see US Patent No. 5,821,333 ). Such variant C_{H}3 domains may be used to make multispecific (e.g. bispecific) antibodies as herein described.

"Hinge region" is generally defined as stretching from about Glu216, or about Cys226, to about Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions. The hinge region herein may be a native sequence hinge region or a variant hinge region. The two polypeptide chains of a variant hinge region generally retain at least one cysteine residue per polypeptide chain, so that the two polypeptide chains of the variant hinge region can form a disulfide bond between the two chains. The preferred hinge region herein is a native sequence human hinge region, e.g. a native sequence human IgG1 hinge region.

A "functional Fc region" possesses at least one "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (e.g. B cell receptor; BCR), etc. Such effector functions generally require the Fc region to be combined with a binding domain (e.g. an antibody variable domain) and can be assessed using various assays known in the art for evaluating such antibody effector functions.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "variant Fc region" comprises an amino acid sequence which differs from that of a native sequence Fc region by virtue of at least one amino acid modification. In certain embodiments, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide, e.g. from about one to about ten amino acid substitutions, and preferably from about one to about five amino acid substitutions in a native sequence Fc region or in the Fc region of the parent polypeptide. The variant Fc region herein will typically possess, e.g., at least about 80% sequence identity with a native sequence Fc region and/or with an Fc region of a parent polypeptide, or at least about 90% sequence identity therewith, or at least about 95% sequence or more identity therewith.

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

"Antibody-dependent cell-mediated cytotoxicity" or "ADCC" refers to a form of cytotoxicity in which secreted Ig bound onto Fc receptors (FcRs) present on certain cytotoxic cells (e.g. Natural Killer (NK) cells, neutrophils, and macrophages) enable these cytotoxic effector cells to bind specifically to an antigen-bearing target cell and subsequently kill the target cell with cytotoxins. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an in vitro ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. PNAS (USA) 95:652-656 (1998).

"Human effector cells" are leukocytes which express one or more FcRs and perform effector functions. In certain embodiments, the cells express at least FcγRIII and perform ADCC effector function(s). Examples of human leukocytes which mediate ADCC include peripheral blood mononuclear cells (PBMC), natural killer (NK) cells, monocytes, cytotoxic T cells and neutrophils; with PBMCs and NK cells being generally preferred. The effector cells may be isolated from a native source thereof, e.g. from blood or PBMCs as described herein.

"Fc receptor" or "FcR" describes a receptor that binds to the Fc region of an antibody. In some embodiments, an FcR is a native human FcR. In some embodiments, an FcR is one which binds an IgG antibody (a gamma receptor) and includes receptors of the FcγRI, FcγRII, and FcγRIII subclasses, including allelic variants and alternatively spliced forms of those receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see, *e.g.,* Dacron. Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, *e.g*., Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton et al.).

Binding to human FcRn *in vivo* and serum half life of human FcRn high affinity binding polypeptides can be assayed, *e.g.*, in transgenic mice or transfected human cell lines expressing human FcRn, or in primates to which the polypeptides with a variant Fc region are administered. WO 2000/42072 (Presta) describes antibody variants with improved or diminished binding to FcRs. See also, *e.g.,* Shields et al. J. Biol. Chem. 9(2):6591-6604 (2001).

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, *e.g.,* as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, e.g., in US Patent No. 6,194,551 B1 and WO 1999/51642. See also, *e.g.*, Idusogie et al. J. Immunol. 164: 4178-4184 (2000).

An "affinity matured" antibody is one with one or more alterations in one or more CDRs thereof which result an improvement in the affinity of the antibody for antigen, compared to a parent antibody which does not possess those alteration(s). In one embodiment, an affinity matured antibody has nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "functional antigen binding site" of an antibody is one which is capable of binding a target antigen. The antigen binding affinity of the antigen binding site is not necessarily as strong as the parent antibody from which the antigen binding site is derived, but the ability to bind antigen must be measurable using any one of a variety of methods known for evaluating antibody binding to an antigen. Moreover, the antigen binding affinity of each of the antigen binding sites of a multivalent antibody herein need not be quantitatively the same. For the multimeric antibodies herein, the number of functional antigen binding sites can be evaluated using ultracentrifugation analysis. According to this method of analysis, different ratios of target antigen to multimeric antibody are combined and the average molecular weight of the complexes is calculated assuming differing numbers of functional binding sites. These theoretical values are compared to the actual experimental values obtained in order to evaluate the number of functional binding sites.

An antibody having a "biological characteristic" of a designated antibody is one which possesses one or more of the biological characteristics of that antibody which distinguish it from other antibodies that bind to the same antigen.

In order to screen for antibodies which bind to an epitope on an antigen bound by an antibody of interest, a routine cross-blocking assay such as that described in Antibodies, A Laboratory Manual, Cold Spring Harbor Laboratory, Ed Harlow and David Lane (1988), can be performed.

The term "antagonist" when used herein refers to a molecule capable of neutralizing, blocking, inhibiting, abrogating, reducing or interfering with the activities of a protein of the invention including its binding to one or more receptors in the case of a ligand or binding to one or more ligands in case of a receptor. Antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of a protein of the invention, and fusions proteins, receptor molecules and derivatives which bind specifically to protein thereby sequestering its binding to its target, antagonist variants of the protein, antisense molecules directed to a protein of the invention, RNA aptamers, and ribozymes against a protein of the invention.

A "blocking" antibody or an "antagonist" antibody is one which inhibits or reduces biological activity of the antigen it binds. Certain blocking antibodies or antagonist antibodies substantially or completely inhibit the biological activity of the antigen

The terms "VEGF" and "VEGF-A" are used interchangeably to refer to the 165-amino acid vascular endothelial cell growth factor and related 121-, 145-, 183-, 189-, and 206- amino acid vascular endothelial cell growth factors, as described by Leung et al. Science, 246:1306 (1989), Houck et al. Mol. Endocrin., 5:1806 (1991), and, Robinson & Stringer, Journal of Cell Science, 144(5):853-865 (2001), together with the naturally occurring allelic and processed forms thereof VEGF-A is part of a gene family including VEGF-B, VEGF-C, VEGF-D, VEGF-E, VEGF-F, and P1GF. VEGF-A primarily binds to two high affinity receptor tyrosine kinases, VEGFR-1 (Flt-1) and VEGFR-2 (Flk-1/KDR), the latter being the major transmitter of vascular endothelial cell mitogenic signals of VEGF-A. The term "VEGF" or "VEGF-A" also refers to VEGFs from non-human species such as mouse, rat, or primate. Sometimes the VEGF from a specific species is indicated by terms such as hVEGF for human VEGF or mVEGF for murine VEGF. The term "VEGF" is also used to refer to truncated forms or fragments of the polypeptide comprising amino acids 8 to 109 or 1 to 109 of the 165-amino acid human vascular endothelial cell growth factor. The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

A "VEGF antagonist" refers to a molecule (peptidyl or non-peptidyl) capable of neutralizing, blocking, inhibiting, abrogating, reducing or interfering with VEGF activities including its binding to one or more VEGF receptors. VEGF antagonists include anti-VEGF antibodies and antigen-binding fragments thereof, receptor molecules and derivatives which bind specifically to VEGF thereby sequestering its binding to one or more receptors (e.g., soluble VEGF receptor proteins, or VEGF binding fragments thereof, or chimeric VEGF receptor proteins), anti-VEGF receptor antibodies and VEGF receptor antagonists such as small molecule inhibitors of the VEGFR tyrosine kinases, and fusions proteins, e.g., VEGF-Trap (Regeneron), VEGF₁₂₁-gelonin (Peregine). VEGF antagonists also include antagonist variants of VEGF, antisense molecules directed to VEGF, RNA aptamers, and ribozymes against VEGF or VEGF receptors. VEGF antagonists useful in the methods of the invention further include peptidyl or non-peptidyl compounds that specifically bind VEGF, such as anti-VEGF antibodies and antigen-binding fragments thereof, polypeptides, or fragments thereof that specifically bind to VEGF; antisense nucleobase oligomers complementary to at least a fragment of a nucleic acid molecule encoding a VEGF polypeptide; small RNAs complementary to at least a fragment of a nucleic acid molecule encoding a VEGF polypeptide; ribozymes that target VEGF; peptibodies to VEGF; and VEGF aptamers. In one embodiment, the VEGF antagonist reduces or inhibits, by at least 10%, 20%, 30%, 40%, 50%, 60%, 70%, 80%, 90% or more, the expression level or biological activity of VEGF. In another embodiment, the VEGF inhibited by the VEGF antagonist is VEGF (8-109), VEGF (1-109), or VEGF₁₆₅.

The term "anti-VEGF antibody" or "an antibody that binds to VEGF" refers to an antibody that is capable of binding to VEGF with sufficient affinity and specificity that the antibody is useful as a diagnostic and/or therapeutic agent in targeting VEGF. For example, the anti-VEGF antibody of the invention can be used as a therapeutic agent in targeting and interfering with diseases or conditions wherein the VEGF activity is involved. *See, e.g.,* U.S. Patents 6,582,959, 6,703,020; WO98/45332; WO 96/30046; WO94/10202, WO2005/044853; ; EP 0666868B1; US Patent Applications 20030206899, 20030190317, 20030203409, 20050112126, 20050186208, and 20050112126; Popkov et al., Journal of Immunological Methods 288:149-164 (2004); and WO2005012359. The antibody selected will normally have a sufficiently strong binding affinity for VEGF. For example, the antibody may bind hVEGF with a K_{d} value of between 100 nM-1 pM. Antibody affinities may be determined by a surface plasmon resonance based assay (such as the BIAcore assay as described in PCT Application Publication No. WO2005/012359); enzyme-linked immunoabsorbent assay (ELISA); and competition assays (e.g. RIA's), for example. The antibody may be subjected to other biological activity assays, e.g., in order to evaluate its effectiveness as a therapeutic. Such assays are known in the art and depend on the target antigen and intended use for the antibody. Examples include the HUVEC inhibition assay; tumor cell growth inhibition assays (as described in WO 89/06692, for example); antibody-dependent cellular cytotoxicity (ADCC) and complement-mediated cytotoxicity (CDC) assays (US Patent 5,500,362); and agonistic activity or hematopoiesis assays (see WO 95/27062). An anti-VEGF antibody will usually not bind to other VEGF homologues such as VEGF-B, VEGF-C, VEGF-D or VEGF-E, nor other growth factors such as P1GF, PDGF or bFGF. In one embodiment, anti-VEGF antibodies include a monoclonal antibody that binds to the same epitope as the monoclonal anti-VEGF antibody A4.6.1 produced by hybridoma ATCC HB 10709; a recombinant humanized anti-VEGF monoclonal antibody (*see* Presta et al. (1997) Cancer Res. 57:4593-4599), including but not limited to the antibody known as "bevacizumab (BV)," also known as "rhuMAb VEGF" or "AVASTIN™." AVASTIN™ is presently commercially available. Bevacizumab comprises mutated human IgG1 framework regions and antigen-binding complementarity-determining regions from the murine antibody A.4.6.1 that blocks binding of human VEGF to its receptors. Approximately 93% of the amino acid sequence of bevacizumab, including most of the framework regions, is derived from human IgG1, and about 7% of the sequence is derived from A4.6.1. Bevacizumab has a molecular mass of about 149,000 daltons and is glycosylated. Bevacizumab and other humanized anti-VEGF antibodies are further described in U.S. Pat. No. 6,884,879 issued February 26, 2005. Additional anti-VEGF antibodies include the G6 or B20 series antibodies (e.g., G6-23, G6-31, B20-4.1), as described in PCT Application Publication No. WO2005/012359. For additional preferred antibodies see U.S. Pat. Nos. 7,060,269, 6,582,959, 6,703,020; 6,054,297; WO98/45332; WO 96/30046; WO94/10202; EP 0666868B1; U.S. Patent Application Publication Nos. 2006009360, 20050186208, 20030206899, 20030190317, 20030203409, and 20050112126; and Popkov et al., Journal of Immunological Methods 288:149-164 (2004).

The term "B20 series polypeptide" as used herein refers to a polypeptide, including an antibody that binds to VEGF. B20 series polypeptides includes, but not limited to, antibodies derived from a sequence of the B20 antibody or a B20-derived antibody described in US Publication No. 20060280747, US Publication No. 20070141065 and/or US Publication No. 20070020267. In one embodiment, B20 series polypeptide is B20-4.1 as described in US Publication No. 20060280747, US Publication No. 20070141065 and/or US Publication No. 20070020267. In another embodiment, B20 series polypeptide is B20-4.1.1 described in PCT Publication No. WO 2009/073160.

The term "G6 series polypeptide" as used herein refers to a polypeptide, including an antibody that binds to VEGF. G6 series polypeptides includes, but not limited to, antibodies derived from a sequence of the G6 antibody or a G6-derived antibody described in US Publication No. 20060280747, US Publication No. 20070141065 and/or US Publication No. 20070020267. G6 series polypeptides, as described in US Publication No. 20060280747, US Publication No. 20070141065 and/or US Publication No. 20070020267 include, but not limited to, G6-8, G6-23 and G6-31.

The term "bFGF", also known as "FGF2", "FGF-β" or "basic fibroblast growth factor", is a member of the fibroblast growth factor family, encoded for by a gene located on the short arm of chromosome 4. The term bFGF as used herein includes a native sequence bFGF polypeptide and bFGF variants. Basic fibroblast growth factor (bFGF) is a soluble heparin binding polypeptide. bFGF also binds to a receptor termed FGFR-1 (Flg). bFGF has a mitogenic effect on endothelial cells and is a potent inducer of angiogenesis. The paracrine production of bFGF in tumor cells has been reported to be associated with the angiogenic switch of tumor development (Kandel, J., et al., Cell, 1991. 66(6): p. 1095-104). Beyond its independent effect on endothelial cells, bFGF also works synergistically with VEGF in inducing angiogenesis (Asahara, T., et al., Circulation, 1995. 92(9 Suppl): p. 11365-71).

Native sequence bFGF comprises a polypeptide having the same amino acid sequence as bFGF derived from nature, regardless of its mode of preparation. Thus, native sequence bFGF can have the amino acid sequence of naturally occurring human bFGF, murine bFGF, or bFGF from any other mammalian species. Human bFGF sequences are also disclosed (SEQ ID NOs: 1 to 5). Such native sequence bFGF can be isolated from nature or can be produced by recombinant and/or synthetic means. The term native sequence bFGF specifically encompasses naturally occurring prepro, pro and mature forms and truncated forms of bFGF, naturally occurring variant forms (e.g. alternatively spliced forms), and naturally occurring allelic variants.

bFGF variants are biologically active bFGF polypeptides having an amino acid sequence which differs from the sequence of a native sequence bFGF polypeptide by virtue of an insertion, deletion, modification and/or substitution of one or more amino acid residues within the native sequence. bFGF variants generally have less than 100% sequence identity with a native sequence bFGF. Ordinarily, however, a biologically active bFGF variant will have an amino acid sequence with at least about 70%, 75%, 80%, 85%, 90%, 95% or about 99% amino acid sequence identity. The bFGF variants include peptide fragments of at least 5 amino acids that retain a biological activity of the corresponding native sequence bFGF polypeptide. bFGF variants also include bFGF polypeptides wherein one or more amino acid residues are added at the N- or C-terminus of, or within, a native bFGF sequence. bFGF variants also include bFGF polypeptides where a number of amino acid residues are deleted and optionally substituted by one or more amino acid residues.

The term "bFGF antagonist" when used herein refers to a molecule which binds to bFGF and inhibits or substantially reduces a biological activity of bFGF. Non-limiting examples of bFGF antagonists include antibodies, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. In one embodiment of the invention, the bFGF antagonist is an antibody, especially an anti-bFGF antibody which binds human bFGF.

The term "biological activity" and "biologically active" with regard to a polypeptide refer to the ability of a molecule to specifically bind to and regulate cellular responses, e.g., proliferation, migration, etc. Cellular responses also include those mediated through a receptor, including, but not limited to, migration, and/or proliferation. In this context, the term "modulate" includes both promotion and inhibition.

"VEGF biological activity" includes binding to any VEGF receptor or any VEGF signaling activity such as regulation of both normal and abnormal angiogenesis and vasculogenesis (Ferrara and Davis-Smyth (1997) Endocrine Rev. 18:4-25; Ferrara (1999) J. Mol. Med. 77:527-543); promoting embryonic vasculogenesis and angiogenesis *(*Carmeliet et al. (1996) Nature 380:435-439; Ferrara et al. (1996) Nature 380:439-442); and modulating the cyclical blood vessel proliferation in the female reproductive tract and for bone growth and cartilage formation *(*Ferrara et al. (1998) Nature Med. 4:336-340; Gerber et al. (1999) Nature Med. 5:623-628). In addition to being an angiogenic factor in angiogenesis and vasculogenesis, VEGF, as a pleiotropic growth factor, exhibits multiple biological effects in other physiological processes, such as endothelial cell survival, vessel permeability and vasodilation, monocyte chemotaxis and calcium influx (Ferrara and Davis-Smyth (1997), *supra* and Cebe-Suarez et al. Cell. Mol. Life Sci. 63:601-615 (2006)). Moreover, recent studies have reported mitogenic effects of VEGF on a few non-endothelial cell types, such as retinal pigment epithelial cells, pancreatic duct cells, and Schwann cells. Guerrin et al. (1995) J. Cell Physiol. 164:385-394; Oberg-Welsh et al. (1997) Mol. Cell. Endocrinol. 126:125-132; Sondell et al. (1999) J. Neurosci. 19:5731-5740.

An "angiogenic factor or agent" is a growth factor which stimulates the development of blood vessels, e.g., promotes angiogenesis, endothelial cell growth, stability of blood vessels, and/or vasculogenesis, etc. For example, angiogenic factors, include, but are not limited to, e.g., VEGF and members of the VEGF family, PIGF, PDGF family, fibroblast growth factor family (FGFs), TIE ligands (Angiopoietins), ephrins, ANGPTL3, ANGPTL4, etc. It would also include factors that accelerate wound healing, such as growth hormone, insulin-like growth factor-I (IGF-I), VIGF, epidermal growth factor (EGF), CTGF and members of its family, and TGF-α and TGF-β. *See, e.g.,* Klagsbrun and D'Amore, Annu. Rev. Physiol., 53:217-39 (1991); Streit and Detmar, Oncogene, 22:3172-3179 (2003); Ferrara & Alitalo, Nature Medicine 5(12):1359-1364 (1999); Tonini et al., Oncogene, 22:6549-6556 (2003) (e.g., Table 1 listing angiogenic factors); and, Sato Int. J. Clin. Oncol., 8:200-206 (2003).

An "anti-angiogenic agent" or "angiogenesis inhibitor" refers to a small molecular weight substance, a polynucleotide, a polypeptide, an isolated protein, a recombinant protein, an antibody, or conjugates or fusion proteins thereof, that inhibits angiogenesis, vasculogenesis, or undesirable vascular permeability, either directly or indirectly. For example, an anti-angiogenic agent is an antibody or other antagonist to an angiogenic agent as defined above, e.g., antibodies to VEGF, antibodies to VEGF receptors, small molecules that block VEGF receptor signaling (e.g., PTK787/ZK2284, SU6668, SUTENT/SU11248 (sunitinib malate), AMG706). Anti-angiogenic agents also include native angiogenesis inhibitors, e.g., angiostatin, endostatin, etc. *See, e.g.,* Klagsbrun and D'Amore, Annu. Rev. Physiol, 53:217-39 (1991); Streit and Detmar, Oncogene, 22:3172-3179 (2003) (e.g., Table 3 listing anti-angiogenic therapy in malignant melanoma); Ferrara & Alitalo, Nature Medicine 5(12):1359-1364 (1999); Tonini et al., Oncogene, 22:6549-6556 (2003) (e.g., Table 2 listing anti-angiogenic factors); and, Sato Int. J. Clin. Oncol., 8:200-206 (2003) (*e.g.*, Table 1 lists anti-angiogenic agents used in clinical trials).

The term "anti-angiogenic therapy" refers to a therapy useful for inhibiting angiogenesis which comprises the administration of at least one anti-angiogenic agent as defined herein. In certain embodiment, the anti-angiogenic therapy comprises administering VEGF antagonist to a subject. In one embodiment, the anti-angiogenic therapy comprises administering VEGF- antagonist as defined here. In one embodiment, the VEGF antagonist is anti-VEGF antibody. In another embodiment, the anti-VEGF antibody is bevacizumab. In certain embodiments, anti-angiogenic therapy comprises administering 7.5 mg/kg of bevacizumab.

The term "immunosuppressive agent" as used herein refers to substances that act to suppress or mask the immune system of the mammal being treated herein. This would include substances that suppress cytokine production, down-regulate or suppress self-antigen expression, or mask the MHC antigens. Examples of such agents include 2-amino-6-aryl-5-substituted pyrimidines *(see* U.S. Pat. No. 4,665,077); nonsteroidal anti-inflammatory drugs (NSAIDs); ganciclovir, tacrolimus, glucocorticoids such as cortisol or aldosterone, anti-inflammatory agents such as a cyclooxygenase inhibitor, a 5-lipoxygenase inhibitor, or a leukotriene receptor antagonist; purine antagonists such as azathioprine or mycophenolate mofetil (MMF); alkylating agents such as cyclophosphamide; bromocryptine; danazol; dapsone; glutaraldehyde (which masks the MHC antigens, as described in U.S. Pat. No. 4,120,649); anti-idiotypic antibodies for MHC antigens and MHC fragments; cyclosporin A; steroids such as corticosteroids or glucocorticosteroids or glucocorticoid analogs, e.g., prednisone, methylprednisolone, and dexamethasone; dihydrofolate reductase inhibitors such as methotrexate (oral or subcutaneous); hydroxycloroquine; sulfasalazine; leflunomide; cytokine or cytokine receptor antibodies including anti-interferon-alpha, -beta, or -gamma antibodies, anti-tumor necrosis factor-alpha antibodies (infliximab or adalimumab), anti-TNF-alpha immunoahesin (etanercept), anti-tumor necrosis factor-beta antibodies, anti-interleukin-2 antibodies and anti-IL-2 receptor antibodies; anti-LFA-1 antibodies, including anti-CD11a and anti-CD18 antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 1990/08187 published Jul. 26, 1990); streptokinase; TGF-beta; streptodornase; RNA or DNA from the host; FK506; RS-61443; deoxyspergualin; rapamycin; T-cell receptor (Cohen et al., U.S. Pat. No. 5,114,721); T-cell-receptor fragments (Offner et al., Science, 251: 430-432 (1991); WO 1990/11294; Ianeway, Nature, 341: 482 (1989); and WO 1991/01133); and T-cell-receptor antibodies (EP 340,109) such as T10B9.

Examples of "nonsteroidal anti-inflammatory drugs" or "NSAIDs" are acetylsalicylic acid, ibuprofen, naproxen, indomethacin, sulindac, tolmetin, including salts and derivatives thereof, *etc.*

The term "cytotoxic agent" as used herein refers to a substance that inhibits or prevents the function of cells and/or causes destruction of cells. The term is intended to include radioactive isotopes (e.g., ²¹¹At, ¹³¹I, ¹²⁵I, ⁹⁰Y, ¹⁸⁶Re, ¹⁸⁸Re, ¹⁵³Sm, ²¹²Bi, ³²P and radioactive isotopes of Lu), chemotherapeutic agents, and toxins such as small molecule toxins or enzymatically active toxins of bacterial, fungal, plant or animal origin, including fragments and/or variants thereof.

A "growth inhibitory agent" when used herein refers to a compound or composition which inhibits growth of a cell in vitro and/or in vivo. Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), TAXOL™, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13.

A "chemotherapeutic agent" is a chemical compound useful in the treatment of cancer. Examples of chemotherapeutic agents include alkylating agents such as thiotepa and CYTOXAN™ cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL™); beta-lapachone; lapachol; colchicines; betulinic acid; a camptothecin (including the synthetic analogue topotecan (HYCAMTIN™), CPT-11 (irinotecan, CAMPTOSAR™), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e. g*., calicheamicin, especially calicheamicin gamma1I and calicheamicin omegaI1 (see, *e.g.,* Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN™, morpholino-doxorubicin, cyanomorpholino-doxorubicin, 2-pyrrolino-doxorubicin, doxorubicin HCl liposome injection (DOXIL™) and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; pemetrexed (ALIMTA™); gemicitabine (GEMZAR™); anti-metabolites such as methotrexate, gemcitabine (GEMZAR™), tegafur (UFTORAL™), capecitabine (XELODA™), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK™ polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE™, FILDESIN™); dacarbazine; mannomustine; mitobronitol; mitolactol; pipobroman; gacytosine; arabinoside ("Ara-C"); thiotepa; taxoids, e.g., paclitaxel (TAXOL™), albumin-engineered nanoparticle formulation of paclitaxel (ABRAXANE™), and docetaxel (TAXOTERE™); chloranbucil; 6-thioguanine; mercaptopurine; methotrexate; platinum analogs such as cisplatin and carboplatin; vinblastine (VELBAN™); platinum; etoposide (VP-16); ifosfamide; mitoxantrone; vincristine (ONCOVIN™); oxaliplatin; leucovovin; vinorelbine (NAVELBINE™); novantrone; edatrexate; daunomycin; aminopterin; ibandronate; topoisomerase inhibitor RFS 2000; difluorometlhylornithine (DMFO); retinoids such as retinoic acid; pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX™ tamoxifen), raloxifene (EVISTA™), droloxifene, 4-hydroxytamoxifen, trioxifene, keoxifene, LY117018, onapristone, and toremifene (FARESTON™); anti-progesterones; estrogen receptor down-regulators (ERDs); agents that function to suppress or shut down the ovaries, for example, leutinizing hormone-releasing hormone (LHRH) agonists such as leuprolide acetate (LUPRON™ and ELIGARD™), goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, megestrol acetate (MEGASE™), exemestane (AROMASIN™), formestanie, fadrozole, vorozole (RIVISOR™), letrozole (FEMARA™), and anastrozole (ARIMIDEX™). In addition, such definition of chemotherapeutic agents includes bisphosphonates such as clodronate (for example, BONEFOS™ or OSTAC™), etidronate (DIDROCAL™), NE-58095, zoledronic acid/zoledronate (ZOMETA™), alendronate (FOSAMAX™), pamidronate (AREDIA™), tiludronate (SKELID™), or risedronate (ACTONEL™); as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE™ vaccine and gene therapy vaccines, for example, ALLOVECTIN™ vaccine, LEUVECTIN™ vaccine, and VAXID™ vaccine; topoisomerase 1 inhibitor (e.g., LURTOTECAN™); rmRH (e.g., ABARELIX™); lapatinib ditosylate (an ErbB-2 and EGFR dual tyrosine kinase small-molecule inhibitor also known as GW572016); COX-2 inhibitors such as celecoxib (CELEBREX™; 4-(5-(4-methylphenyl)-3-(trifluoromethyl)-1H-pyrazol-1-yl) benzenesulfonamide; and pharmaceutically acceptable salts, acids or derivatives of any of the above.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and -beta; mullerian-inhibiting substance; mouse gonadotropin-associated peptide; inhibin; activin; vascular endothelial growth factors (e.g., VEGF, VEGF-B, VEGF-C, VEGF-D, VEGF-E); placental derived growth factor (P1GF); platelet derived growth factors (PDGF, e.g., PDGFA, PDGFB, PDGFC, PDGFD); integrin; thrombopoietin (TPO); nerve growth factors such as NGF-alpha; platelet-growth factor; transforming growth factors (TGFs) such as TGF-alpha and TGF-beta; insulin-like growth factor-I and -II; erythropoietin (EPO); osteoinductive factors; interferons such as interferon-alpha, -beta and -gamma, colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-1beta, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20-IL-30; secretoglobin/uteroglobin; oncostatin M (OSM); a tumor necrosis factor such as TNF-alpha or TNF-beta; and other polypeptide factors including LIF and kit ligand (KL). As used herein, the term cytokine includes proteins from natural sources or from recombinant cell culture and biologically active equivalents of the native sequence cytokines.

A "disorder" is any condition that would benefit from treatment. This includes chronic and acute disorders or diseases including those pathological conditions which predispose the mammal to the disorder in question. Non-limiting examples of disorders to be treated herein include any form of tumor, benign and malignant tumors; vascularized tumors; hypertrophy; leukemias and lymphoid malignancies; neuronal, glial, astrocytal, hypothalamic and other glandular, macrophagal, epithelial, stromal and blastocoelic disorders; and inflammatory, angiogenic and immunologic disorders, vascular disorders that result from the inappropriate, aberrant, excessive and/or pathological vascularization and/or vascular permeability.

As used herein, "treatment" (and variations such as "treat" or "treating") refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, methods and compositions of the invention are used to delay development of a disease or disorder or to slow the progression of a disease or disorder.

The term "effective amount" or "therapeutically effective amount" refers to an amount of a drug effective to treat a disease or disorder in a mammal. In the case of cancer, the effective amount of the drug may reduce the number of cancer cells; reduce the tumor size; inhibit (i.e., slow to some extent and typically stop) cancer cell infiltration into peripheral organs; inhibit (i.e., slow to some extent and typically stop) tumor metastasis; inhibit, to some extent, tumor growth; allow for treatment of the VEGF-independent tumor, and/or relieve to some extent one or more of the symptoms associated with the disorder. To the extent the drug may prevent growth and/or kill existing cancer cells, it may be cytostatic and/or cytotoxic. For cancer therapy, efficacy *in vivo* can, for example, be measured by assessing the duration of survival, time to disease progression (TTP), the response rates (RR), duration of response, and/or quality of life.

A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount would be less than the therapeutically effective amount.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia or lymphoid malignancies. More particular examples of such cancers include kidney or renal cancer, breast cancer, colon cancer, rectal cancer, colorectal cancer, lung cancer including small-cell lung cancer, non-small cell lung cancer, adenocarcinoma of the lung and squamous carcinoma of the lung, squamous cell cancer (e.g. epithelial squamous cell cancer), cervical cancer, ovarian cancer, prostate cancer, liver cancer, bladder cancer, cancer of the peritoneum, hepatocellular cancer, gastric or stomach cancer including gastrointestinal cancer, gastrointestinal stromal tumors (GIST), pancreatic cancer, head and neck cancer, glioblastoma, retinoblastoma, astrocytoma, thecomas, arrhenoblastomas, hepatoma, hematologic malignancies including non-Hodgkins lymphoma (NHL), multiple myeloma and acute hematologic malignancies, endometrial or uterine carcinoma, endometriosis, fibrosarcomas, choriocarcinoma, salivary gland carcinoma, vulval cancer, thyroid cancer, esophageal carcinomas, hepatic carcinoma, anal carcinoma, penile carcinoma, nasopharyngeal carcinoma, laryngeal carcinomas, Kaposi's sarcoma, melanoma, skin carcinomas, Schwannoma, oligodendroglioma, neuroblastomas, rhabdomyosarcoma, osteogenic sarcoma, leiomyosarcomas, urinary tract carcinomas, thyroid carcinomas, Wilm's tumor, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

"Tumor", as used herein, refers to all neoplastic cell growth and proliferation, whether malignant or benign, and all pre-cancerous and cancerous cells and tissues.

Examples of neoplastic disorders to be treated include, but are not limited to, those described herein under the terms "cancer" and "cancerous."

The term "anti-cancer therapy" or "cancer therapy" refers to a therapy useful in treating cancer. Examples of anti-cancer therapeutic agents include, but are limited to, e.g., chemotherapeutic agents, growth inhibitory agents, cytotoxic agents, agents used in radiation therapy, anti-angiogenic agents, apoptotic agents, anti-tubulin agents, and other agents to treat cancer, such as anti-HER-2 antibodies, anti-CD20 antibodies, an epidermal growth factor receptor (EGFR) antagonist (e.g., a tyrosine kinase inhibitor), HER1/EGFR inhibitor (e.g., erlotinib (TARCEVA™), platelet derived growth factor inhibitors (e.g., GLEEVEC™ (Imatinib Mesylate)), a COX-2 inhibitor (e.g., celecoxib), ERBITUX™ (cetuximab, Imclone), interferons, cytokines, antagonists (e.g., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also included in the invention.

The term "diagnosis" is used herein to refer to the identification of a molecular or pathological state, disease or condition, such as the identification of cancer or to refer to identification of a cancer patient who may benefit from a particular treatment regimen.

The term "prognosis" is used herein to refer to the prediction of the likelihood of clinical benefit from anti-cancer therapy.

The term "prediction" is used herein to refer to the likelihood that a patient will respond favorably to a particular anti-cancer therapy. The predictive methods of the invention can be used clinically to make treatment decisions by choosing the most appropriate treatment modalities for any particular patient. The predictive methods of the present invention are valuable tools in predicting if a patient is likely to respond favorably to a treatment regimen, such as a given therapeutic regimen, including for example, administration of a given therapeutic agent or combination, surgical intervention, steroid treatment, etc.

Responsiveness of a patient can be assessed using any endpoint indicating a benefit to the patient, including, without limitation, (1) inhibition, to some extent, of disease progression, including slowing down and complete arrest; (2) reduction in lesion size; (3) inhibition (i.e., reduction, slowing down or complete stopping) of disease cell infiltration into adjacent peripheral organs and/or tissues; (4) inhibition (i.e. reduction, slowing down or complete stopping) of disease spread; (5) relief, to some extent, of one or more symptoms associated with the disorder; (6) increase in the length of disease-free presentation following treatment; and/or (8) decreased mortality at a given point of time following treatment.

Clinical benefit can be measured by assessing various endpoints, e.g., inhibition, to some extent, of disease progression, including slowing down and complete arrest; reduction in the number of disease episodes and/or symptoms; reduction in lesion size; inhibition *(i.e.,* reduction, slowing down or complete stopping) of disease cell infiltration into adjacent peripheral organs and/or tissues; inhibition (i.e. reduction, slowing down or complete stopping) of disease spread; decrease of auto-immune response, which may, but does not have to, result in the regression or ablation of the disease lesion; relief, to some extent, of one or more symptoms associated with the disorder; increase in the length of disease-free presentation following treatment, e.g., progression-free survival; increased overall survival; higher response rate; and/or decreased mortality at a given point of time following treatment.

The term "benefit" is used in the broadest sense and refers to any desirable effect and specifically includes clinical benefit as defined herein.

The term "pharmaceutical formulation" refers to a preparation which is in such form as to permit the biological activity of the active ingredient to be effective, and which contains no additional components which are unacceptably toxic to a subject to which the formulation would be administered. Such formulations may be sterile.

A "sterile" formulation is aseptic or free from all living microorganisms and their spores.

Administration "in combination with" one or more further therapeutic agents includes simultaneous (concurrent) and consecutive or sequential administration in any order.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

"Chronic" administration refers to administration of the agent(s) in a continuous mode as opposed to an acute mode, so as to maintain the initial therapeutic effect (activity) for an extended period of time.

"Intermittent" administration is treatment that is not consecutively done without interruption, but rather is cyclic in nature.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

A "liposome" is a small vesicle composed of various types of lipids, phospholipids and/or surfactant which is useful for delivery of a drug (such as a anti-VEGF antibody) to a mammal. The components of the liposome are commonly arranged in a bilayer formation, similar to the lipid arrangement of biological membranes.

Anti-angiogenic agents include, but are not limited to, the following agents: VEGF inhibitors such as a VEGF-specific antagonist, EGF inhibitor, EGFR inhibitors, ERBITUX™ (cetuximab, ImClone Systems, Inc., Branchburg, N.J.), VECTIBIX™ (panitumumab, Amgen, Thousand Oaks, CA), TIE2 inhibitors, IGF1R inhibitors, COX-II (cyclooxygenase II) inhibitors, MMP-2 (matrix-metalloprotienase 2) inhibitors, and MMP-9 (matrix-metalloprotienase 9) inhibitors, CP-547,632 (Pfizer Inc., NY, USA), Axitinib (Pfizer Inc.; AG-013736), ZD-6474 (AstraZeneca), AEE788 (Novartis), AZD-2171), VEGF Trap (Regeneron/Aventis), Vatalanib (also known as PTK-787, ZK-222584: Novartis & Schering AG), Macugen (pegaptanib octasodium, NX-1838, EYE-001, Pfizer Inc./Gilead/Eyetech), IM862 (Cytran Inc. of Kirkland, Wash., USA); and angiozyme, a synthetic ribozyme from Ribozyme (Boulder, Colo.) and Chiron (Emeryville, Calif.) and combinations thereof. Other angiogenesis inhibitors include thrombospondin1, thrombospondin2, collagen IV and collagen XVIII. VEGF inhibitors are disclosed in U.S. Pat. Nos. 6,534,524 and 6,235,764.

A VEGF-specific antagonist refers to a molecule capable of binding to VEGF, reducing VEGF expression levels, or neutralizing, blocking, inhibiting, abrogating, reducing, or interfering with VEGF biological activities, including VEGF binding to one or more VEGF receptors and VEGF mediated angiogenesis and endothelial cell survival or proliferation. Included as VEGF-specific antagonists useful in the methods of the invention are polypeptides that specifically bind to VEGF, anti-VEGF antibodies and antigen-binding fragments thereof, receptor molecules and derivatives which bind specifically to VEGF thereby sequestering its binding to one or more receptors, fusions proteins (e.g., VEGF-Trap (Regeneron)), and VEGF₁₂₁-gelonin (Peregrine). VEGF-specific antagonists also include antagonist variants of VEGF polypeptides, antisense nucleobase oligomers directed to VEGF, small RNA molecules directed to VEGF, RNA aptamers, peptibodies, and ribozymes against VEGF.

The two best characterized VEGF receptors are VEGFR1 (also known as Flt-1) and VEGFR2 (also known as KDR and FLK-1 for the murine homolog). The specificity of each receptor for each VEGF family member varies but VEGF-A binds to both Flt-1 and KDR. The full length Flt-1 receptor includes an extracellular domain that has seven Ig domains, a transmembrane domain, and an intracellular domain with tyrosine kinase activity. The extracellular domain is involved in the binding of VEGF and the intracellular domain is involved in signal transduction.

VEGF receptor molecules, or fragments thereof, that specifically bind to VEGF can be used as VEGF inhibitors that bind to and sequester the VEGF protein, thereby preventing it from signaling. In certain embodiments, the VEGF receptor molecule, or VEGF binding fragment thereof, is a soluble form, such as sFlt-1. A soluble form of the receptor exerts an inhibitory effect on the biological activity of the VEGF protein by binding to VEGF, thereby preventing it from binding to its natural receptors present on the surface of target cells. Also included are VEGF receptor fusion proteins, examples of which are described below.

A chimeric VEGF receptor protein is a receptor molecule having amino acid sequences derived from at least two different proteins, at least one of which is a VEGF receptor protein (e.g., the flt-1 or KDR receptor), that is capable of binding to and inhibiting the biological activity of VEGF. In certain embodiments, the chimeric VEGF receptor proteins of the present invention consist of amino acid sequences derived from only two different VEGF receptor molecules; however, amino acid sequences comprising one, two, three, four, five, six, or all seven Ig-like domains from the extracellular ligand-binding region of the flt-1 and/or KDR receptor can be linked to amino acid sequences from other unrelated proteins, for example, immunoglobulin sequences. Other amino acid sequences to which Ig-like domains are combined will be readily apparent to those of ordinary skill in the art. Examples of chimeric VEGF receptor proteins include, but not limited to, soluble Flt-1/Fc, KDR/Fc, or Flt-1/KDR/Fc (also known as VEGF Trap). *(See* for example PCT Application Publication No. WO97/44453).

A soluble VEGF receptor protein or chimeric VEGF receptor proteins includes VEGF receptor proteins which are not fixed to the surface of cells via a transmembrane domain. As such, soluble forms of the VEGF receptor, including chimeric receptor proteins, while capable of binding to and inactivating VEGF, do not comprise a transmembrane domain and thus generally do not become associated with the cell membrane of cells in which the molecule is expressed.

Additional VEGF inhibitors are described in, for example in WO 99/24440, PCT International Application PCT/IB99/00797, in WO 95/21613, WO 99/61422, U.S. Pat. No. 6,534,524, U.S. Pat. No. 5,834,504, WO 98/50356, U.S. Pat. No. 5,883,113, U.S. Pat. No. 5,886,020, U.S. Pat. No. 5,792,783, U.S. Pat. No. 6,653,308, WO 99/10349, WO 97/32856, WO 97/22596, WO 98/54093, WO 98/02438, WO 99/16755, and WO 98/02437.

Overall, 53-74% of patients with NSCLC have been reported to have bFGF expressed in tumor tissue specimens (Takanami, I., et al., Jpn J Clin Oncol, 1996.26(5): p. 293-7; Volm, M., et al., Eur J Cancer, 1997. 33(4): p. 691-3; Shou, Y., et al., Br J Cancer, 2001. 85(11): p. 1706-12) and bFGF expression was associated with higher T and N (in the TNM classification); higher tumor stage; and poor survival (Takanami, I., et al., Jpn J Clin Oncol, 1996. 26(5): p. 293-7). bFGF expression has also been reported to be associated with low differentiation, vessel invasion, and lymph node metastasis in patients with NSCLC (Volm, M., et al., Anticancer Res, 1999. 19(1B): p. 651-5; Ito, H., et al., Oncol Rep, 2002. 9(1): p. 119-23).

Studies examining the role of circulating bFGF in patients with NSCLC have shown variable results, from a favorable association between increased bFGF levels and survival (Brattstrom, D., et al., Anticancer Res, 1998. 18(2A): p. 1123-7), no correlation with survival (Ueno, K., et al., Lung Cancer, 2001. 31(2-3): p. 213-9) and a negative association between elevated levels and survival (Brattstrom, D., et al., Lung Cancer, 2002. 37(1): p. 57-63; Brattstrom, D., et al., Lung Cancer, 2004. 43(1): p. 55-62; Joensuu, H., et al., Cancer Res, 2002. 62(18): p. 5210-7).

Circulating bFGF has also been suggested to be associated with the escape of tumors from anti-VEGF therapy, since up-regulation of alternative pro-angiogenic signaling pathways (such as bFGF) may be involved in resistance to anti-VEGF therapy (Jain, R.K., et al., Nat Rev Clin Oncol, 2009. 6(6): p. 327-38; Dempke, W.C. and V. Heinemann, Eur J Cancer, 2009. 45(7): p. 1117-28).

The present invention is based partly on the identification that expression level of bFGF is useful in predicting and/or monitoring the progress of anti-angiogenic therapy comprising an anti-VEGF antibody bevacizumab. In particular, the present invention is based partly on the identification that bFGF protein level in blood is useful in predicting patient responsiveness to an anti-VEGF antibody therapy. Thus, the disclosed methods and assays provide convenient, efficient, and potentially cost-effective means to obtain data and information useful in assessing appropriate or effective therapies for treating cancer patients. For example, a sample from a cancer patient could be examined by various in vitro assays to determine whether the expression level of bFGF in a sample is higher than the expression level of bFGF in a reference sample. If a higher expression level is detected, the patient will probably benefit from anti-cancer therapy comprising anti-VEGF antibody. In certain embodiments, if a higher expression level of bFGF is detected, the patient will probably benefit from anti-angiogenic therapy comprising administration of 7.5 mg/kg of anti-VEGF antibody bevacizumab.

Expression levels/amount of a biomarker can be determined based on any suitable criterion known in the art, including but not limited to mRNA, cDNA, proteins, protein fragments and/or gene copy number.

For example, expression level of bFGF in a sample can be analyzed by a number of methodologies, many of which are known in the art and understood by the skilled artisan, including but not limited to, immunohistochemical and/or Western blot analysis, immuno-precipitation, molecular binding assays, ELISA, ELIFA, fluorescence activated cell sorting (FACS) and the like, quantitative blood based assays (as for example Serum ELISA) (to examine, for example, levels of protein expression), biochemical enzymatic activity assays, *in situ* hybridization, Northern analysis and/or PCR analysis of mRNAs, as well as any one of the wide variety of assays that can be performed by gene and/or tissue array analysis. Typical protocols for evaluating the status of genes and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery (MSD) may also be used.

A sample comprising bFGF can be obtained by methods well known in the art, and that are appropriate for the particular type and location of the cancer of interest. *See* under Definitions. For instance, samples of cancerous lesions may be obtained by resection, bronchoscopy, fine needle aspiration, bronchial brushings, or from sputum, pleural fluid or blood. Genes or gene products, e.g., bFGF gene, bFGF protein or bFGF mRNA, can be detected from cancer or tumor tissue or from other body samples such as urine, sputum, serum or plasma. The same techniques discussed above for detection of target genes or gene products in cancerous samples can be applied to other body samples. Cancer cells may be sloughed off from cancer lesions and appear in such body samples. By screening such body samples, a simple early diagnosis can be achieved for these cancers. In addition, the progress of therapy can be monitored more easily by testing such body samples for target genes or gene products.

Means for enriching a tissue preparation for cancer cells are known in the art. For example, the tissue may be isolated from paraffin or cryostat sections. Cancer cells may also be separated from normal cells by flow cytometry or laser capture microdissection. These, as well as other techniques for separating cancerous from normal cells, are well known in the art. If the cancer tissue is highly contaminated with normal cells, detection of signature gene or protein expression profile may be more difficult, although techniques for minimizing contamination and/or false positive/negative results are known, some of which are described herein below. For example, a sample may also be assessed for the presence of a biomarker known to be associated with a cancer cell of interest but not a corresponding normal cell, or vice versa.

In certain embodiments, the expression of proteins in a sample is examined using immunohistochemistry ("IHC") and staining protocols. Immunohistochemical staining of tissue sections has been shown to be a reliable method of assessing or detecting presence of proteins in a sample. Immunohistochemistry techniques utilize an antibody to probe and visualize cellular antigens *in situ,* generally by chromogenic or fluorescent methods.

The tissue sample may be fixed *(i.e.* preserved) by conventional methodology (See *e.g.,* "Manual of Histological Staining Method of the Armed Forces Institute of Pathology," 3rd edition (1960) Lee G. Luna, HT (ASCP) Editor, The Blakston Division McGraw-Hill Book Company, New York; The Armed Forces Institute of Pathology Advanced Laboratory Methods in Histology and Pathology (1994) Ulreka V. Mikel, Editor, Armed Forces Institute of Pathology, American Registry of Pathology, Washington, D.C.). One of skill in the art will appreciate that the choice of a fixative is determined by the purpose for which the sample is to be histologically stained or otherwise analyzed. One of skill in the art will also appreciate that the length of fixation depends upon the size of the tissue sample and the fixative used. By way of example, neutral buffered formalin, Bouin's or paraformaldehyde, may be used to fix a sample.

Generally, the sample is first fixed and is then dehydrated through an ascending series of alcohols, infiltrated and embedded with paraffin or other sectioning media so that the tissue sample may be sectioned. Alternatively, one may section the tissue and fix the sections obtained. By way of example, the tissue sample may be embedded and processed in paraffin by conventional methodology (See ***e.g.,*** "Manual of Histological Staining Method of the Armed Forces Institute of Pathology", *sura*). Examples of paraffin that may be used include, but are not limited to, Paraplast, Broloid, and Tissuemay. Once the tissue sample is embedded, the sample may be sectioned by a microtome or the like (See ***e.g.,*** "Manual of Histological Staining Method of the Armed Forces Institute of Pathology", *sura*). By way of example for this procedure, sections may range from about three microns to about five microns in thickness. Once sectioned, the sections may be attached to slides by several standard methods. Examples of slide adhesives include, but are not limited to, silane, gelatin, poly-L-lysine and the like. By way of example, the paraffin embedded sections may be attached to positively charged slides and/or slides coated with poly-L-lysine.

If paraffin has been used as the embedding material, the tissue sections are generally deparaffinized and rehydrated to water. The tissue sections may be deparaffinized by several conventional standard methodologies. For example, xylenes and a gradually descending series of alcohols may be used *(See e.g.*, "Manual of Histological Staining Method of the Armed _{'} Forces Institute of Pathology", *sura*). Alternatively, commercially available deparaffinizing non-organic agents such as Hemo-De7 (CMS, Houston, Texas) may be used.

In certain embodiments, subsequent to the sample preparation, a tissue section may be analyzed using IHC. IHC may be performed in combination with additional techniques such as morphological staining and/or fluorescence *in*-*situ* hybridization. Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for immunohistochemistry typically will be labeled with a detectable moiety. Numerous labels are available which can be generally grouped into the following categories:
(a) Radioisotopes, such as ³⁵S, ¹⁴C, ¹²⁵I, ³H, and ¹³¹I. The antibody can be labeled with the radioisotope using the techniques described in Current Protocols in Immunology, Volumes 1 and 2, Coligen et al., Ed. Wiley-Interscience, New York, New York, Pubs. (1991) for example and radioactivity can be measured using scintillation counting.
(b) Colloidal gold particles.
(c) Fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above. The fluorescent labels can be conjugated to the antibody using the techniques disclosed in *Current Protocols in Immunology, supra*, for example. Fluorescence can be quantified using a fluorimeter.
(d) Various enzyme-substrate labels are available and U.S. Patent No. 4,275,149 provides a review of some of these. The enzyme generally catalyzes a chemical alteration of the chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (*e.g.*, firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed. J. Langone & H. Van Vunakis), Academic press, New York, 73:147-166 (1981).\

Examples of enzyme-substrate combinations include, for example:
(i) Horseradish peroxidase (HRPO) with hydrogen peroxidase as a substrate, wherein the hydrogen peroxidase oxidizes a dye precursor (e.g., orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate ***(e.g.,*** p-nitrophenyl-β-D-galactosidase) or fluorogenic substrate ***(e.g.,*** 4-methylumbelliferyl-β-D-galactosidase).

Numerous other enzyme-substrate combinations are available to those skilled in the art. For a general review of these, see U.S. Patent Nos. 4,275,149 and 4,318,980. Sometimes, the label is indirectly conjugated with the antibody. The skilled artisan will be aware of various techniques for achieving this. For example, the antibody can be conjugated with biotin and any of the four broad categories of labels mentioned above can be conjugated with avidin, or *vice versa.* Biotin binds selectively to avidin and thus, the label can be conjugated with the antibody in this indirect manner. Alternatively, to achieve indirect conjugation of the label with the antibody, the antibody is conjugated with a small hapten and one of the different types of labels mentioned above is conjugated with an anti-hapten antibody. Thus, indirect conjugation of the label with the antibody can be achieved.

Aside from the sample preparation procedures discussed above, further treatment of the tissue section prior to, during or following IHC may be desired. For example, epitope retrieval methods, such as heating the tissue sample in citrate buffer may be carried out *(see, e.g.,* Leong et al. Appl. Immunohistochem. 4(3):201 (1996)).

Following an optional blocking step, the tissue section is exposed to primary antibody for a sufficient period of time and under suitable conditions such that the primary antibody binds to the target protein antigen in the tissue sample. Appropriate conditions for achieving this can be determined by routine experimentation. The extent of binding of antibody to the sample is determined by using any one of the detectable labels discussed above. In certain embodiments, the label is an enzymatic label ***(e.g.*** HRPO) which catalyzes a chemical alteration of the chromogenic substrate such as 3,3'-diaminobenzidine chromogen. In one embodiment, the enzymatic label is conjugated to antibody which binds specifically to the primary antibody ***(e.g.*** the primary antibody is rabbit polyclonal antibody and secondary antibody is goat anti-rabbit antibody).

Specimens thus prepared may be mounted and coverslipped. Slide evaluation is then determined, ***e.g.,*** using a microscope, and staining intensity criteria, routinely used in the art, may be employed. Staining intensity criteria may be evaluated as follows:

| **Staining Pattern** | **Score** |
|---|---|
| No staining is observed in cells. | 0 |
| Faint/barely perceptible staining is detected in more than 10% of the cells. | 1+ |
| Weak to moderate staining is observed in more than 10% of the cells. | 2+ |
| Moderate to strong staining is observed in more than 10% of the cells. | 3+ |

In alternative methods, the sample may be contacted with an antibody specific for said biomarker, e.g., bFGF, under conditions sufficient for an antibody-biomarker complex to form, and then detecting said complex. The presence of the biomarker may be detected in a number of ways, such as by Western blotting and ELISA procedures for assaying a wide variety of tissues and samples, including plasma or serum. A wide range of immunoassay techniques using such an assay format are available, see, e.g., U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used assays. A number of variations of the sandwich assay technique exist, and all are intended to be encompassed by the present invention. Briefly, in a typical forward assay, an unlabelled antibody is immobilized on a solid substrate, and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of biomarker.

Variations on the forward assay include a simultaneous assay, in which both sample and labeled antibody are added simultaneously to the bound antibody. These techniques are well known to those skilled in the art, including any minor variations as will be readily apparent. In a typical forward sandwich assay, a first antibody having specificity for the biomarker is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to 40°C such as between 25° C and 32° C inclusive) to allow binding of any subunit present in the antibody. Following the incubation period, the antibody subunit solid phase is washed and dried and incubated with a second antibody specific for a portion of the biomarker. The second antibody is linked to a reporter molecule which is used to indicate the binding of the second antibody to the molecular marker.

An alternative method involves immobilizing the target biomarkers in the sample and then exposing the immobilized target to specific antibody which may or may not be labeled with a reporter molecule. Depending on the amount of target and the strength of the reporter molecule signal, a bound target may be detectable by direct labeling with the antibody. Alternatively, a second labeled antibody, specific to the first antibody is exposed to the target-first antibody complex to form a target-first antibody-second antibody tertiary complex. The complex is detected by the signal emitted by the reporter molecule. By "reporter molecule", as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of biomarker which was present in the sample. Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic color visually detectable with a light microscope. As in the EIA, the fluorescent labeled antibody is allowed to bind to the first antibody-molecular marker complex. After washing off the unbound reagent, the remaining tertiary complex is then exposed to the light of the appropriate wavelength, the fluorescence observed indicates the presence of the molecular marker of interest. Immunofluorescence and EIA techniques are both very well established in the art. However, other reporter molecules, such as radioisotope, chemiluminescent or bioluminescent molecules, may also be employed.

It is contemplated that the above described techniques may be employed to detect protein expression level of bFGF.

Methods of the invention further include protocols which examine the mRNA expression level of bFGF in a sample. Methods for the evaluation of mRNAs in cells are well known and include, for example, hybridization assays using complementary DNA probes (such *as in situ* hybridization using labeled riboprobes specific for the one or more genes, Northern blot and related techniques) and various nucleic acid amplification assays (such as RT-PCR using complementary primers specific for one or more of the genes, and other amplification type detection methods, such as, for example, branched DNA, SISBA, TMA and the like).

In certain embodiments, tissue or cell samples from mammals can be conveniently assayed for mRNAs using Northern, dot blot or PCR analysis. For example, RT-PCR assays such as quantitative PCR assays are well known in the art. In an illustrative embodiment of the invention, a method for detecting a target mRNA in a biological sample comprises producing cDNA from the sample by reverse transcription using at least one primer; amplifying the cDNA so produced using a target polynucleotide as sense and antisense primers to amplify target cDNAs therein; and detecting the presence of the amplified target cDNA using polynucleotide probes. In addition, such methods can include one or more steps that allow one to determine the levels of target mRNA in a biological sample *(e.g.,* by simultaneously examining the levels a comparative control mRNA sequence of a "housekeeping" gene such as an actin family member). Optionally, the sequence of the amplified target cDNA can be determined.

Optional methods of the invention include protocols which examine or detect mRNAs, such as target mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. Hybridization of a labeled probe with a particular array member indicates that the sample from which the probe was derived expresses that gene. Differential gene expression analysis of disease tissue can provide valuable information. Microarray technology utilizes nucleic acid hybridization techniques and computing technology to evaluate the mRNA expression profile of thousands of genes within a single experiment (*see, e.g.,* WO 01/75166 published October 11, 2001; U.S. 5,700,637; U.S. Patent 5,445,934; U.S. Patent 5,807,522; Lockart, Nature Biotechnology, 14:1675-1680 (1996); Cheung, V.G. et al., Nature Genetics 21(Suppl):15-19 (1999) for a discussion of array fabrication). DNA microarrays are miniature arrays containing gene fragments that are either synthesized directly onto or spotted onto glass or other substrates. Thousands of genes are usually represented in a single array. A typical microarray experiment involves the following steps: 1) preparation of fluorescently labeled target from RNA isolated from the sample, 2) hybridization of the labeled target to the microarray, 3) washing, staining, and scanning of the array, 4) analysis of the scanned image and 5) generation of gene expression profiles. Currently two main types of DNA microarrays are being used: oligonucleotide (usually 25 to 70 mers) arrays and gene expression arrays containing PCR products prepared from cDNAs. In forming an array, oligonucleotides can be either prefabricated and spotted to the surface or directly synthesized on to the surface (in situ).

The Affymetrix GeneChip® system is a commercially available microarray system which comprises arrays fabricated by direct synthesis of oligonucleotides on a glass surface. Probe/Gene Arrays: Oligonucleotides, usually 25 mers, are directly synthesized onto a glass wafer by a combination of semiconductor-based photolithography and solid phase chemical synthesis technologies. Each array contains up to 400,000 different oligos and each oligo is present in millions of copies. Since oligonucleotide probes are synthesized in known locations on the array, the hybridization patterns and signal intensities can be interpreted in terms of gene identity and relative expression levels by the Affymetrix Microarray Suite software. Each gene is represented on the array by a series of different oligonucleotide probes. Each probe pair consists, of a perfect match oligonucleotide and a mismatch oligonucleotide. The perfect match probe has a sequence exactly complimentary to the particular gene and thus measures the expression of the gene. The mismatch probe differs from the perfect match probe by a single base substitution at the center base position, disturbing the binding of the target gene transcript. This helps to determine the background and nonspecific hybridization that contributes to the signal measured for the perfect match oligo. The Microarray Suite software subtracts the hybridization intensities of the mismatch probes from those of the perfect match probes to determine the absolute or specific intensity value for each probe set. Probes are chosen based on current information from Genbank and other nucleotide repositories. The sequences are believed to recognize unique regions of the 3' end of the gene. A GeneChip Hybridization Oven ("rotisserie" oven) is used to carry out the hybridization of up to 64 arrays at one time. The fluidics station performs washing and staining of the probe arrays. It is completely automated and contains four modules, with each module holding one probe array. Each module is controlled independently through Microarray Suite software using preprogrammed fluidics protocols. The scanner is a confocal laser fluorescence scanner which measures fluorescence intensity emitted by the labeled cRNA bound to the probe arrays. The computer workstation with Microarray Suite software controls the fluidics station and the scanner. Microarray Suite software can control up to eight fluidics stations using preprogrammed hybridization, wash, and stain protocols for the probe array. The software also acquires and converts hybridization intensity data into a presence/absence call for each gene using appropriate algorithms. Finally, the software detects changes in gene expression between experiments by comparison analysis and formats the output into .txt files, which can be used with other software programs for further data analysis.

Expression of a biomarker in a tissue or cell sample may also be examined by way of functional or activity-based assays. For instance, if the biomarker is an enzyme, one may conduct assays known in the art to determine or detect the presence of the given enzymatic activity in the tissue or cell sample.

The kits of the disclosure have a number of embodiments. In certain embodiments, a kit comprises a container, a label on said container, and a composition contained within said container; wherein the composition includes one or more primary antibodies that bind to bFGF, the label on the container indicating that the composition can be used to evaluate the presence of bFGF protein in at least one type of mammalian cell, and instructions for using the antibodies for evaluating the presence of bFGF protein in at least one type of mammalian cell. The kit can further comprise a set of instructions and materials for preparing a tissue sample and applying antibody and probe to the same section of a tissue sample. The kit may include both a primary and secondary antibody, wherein the secondary antibody is conjugated to a label, *e.g.,* an enzymatic label.

Another embodiment is a kit comprising a container, a label on said container, and a composition contained within said container; wherein the composition includes one or more polynucleotides that hybridize to the polynucleotide sequence of bFGF, under stringent conditions, the label on said container indicates that the composition can be used to evaluate the presence of and/or expression levels of bFGF in at least one type of mammalian cell, and instructions for using the polynucleotide for evaluating the presence of and/or expression levels of bFGF RNAs or DNAs in at least one type of mammalian cell.

Other optional components in the kit include one or more buffers *(e.g.,* block buffer, wash buffer, substrate buffer, etc), other reagents such as substrate *(e.g.,* chromogen) which is chemically altered by an enzymatic label, epitope retrieval solution, control samples (positive and/or negative controls), control slide(s) etc.

The present disclosure contemplates a method for treating an angiogenic disorder (e.g., a disorder characterized by abnormal angiogenesis or abnormal vascular leakage) in a patient comprising the steps of determining that a sample obtained from the patient has higher expression level ofbFGF as compared to a reference sample, and administering to the patient an effective amount of an anti-angiogenic agent whereby the tumor, cancer or cell proliferative disorder is treated. In certain embodiments the anti-angiogenic therapy comprises administering a VEGF antagonist. In certain embodiment, the VEGF antagonist is an anti-VEGF antibody. In certain embodiments, the anti-VEGF antibody is bevacizumab. In certain embodiments, the methods comprise administering 5 mg/kg, 7.5 mg/kg, 10 mg/kg or 15 mg/kg of bevacizumab to the patient having higher expression level of bFGF.

Examples of angiogenic disorders to be treated herein include, but are not limited to cancer, especially vascularized solid tumors and metastatic tumors (including colon cancer, lung cancer, breast cancer, ovarian cancer, renal cancer and glioblastoma), diseases caused by ocular neovascularisation, especially diabetic blindness, retinopathies, primarily diabetic retinopathy or age-related macular degeneration, choroidal neovascularization (CNV), diabetic macular edema, pathological myopia, von Hippel-Lindau disease, histoplasmosis of the eye, Central Retinal Vein Occlusion (CRVO), corneal neovascularization, retinal neovascularization and rubeosis; psoriasis, psoriatic arthritis, haemangioblastoma such as haemangioma; inflammatory renal diseases, such as glomerulonephritis, especially mesangioproliferative glomerulonephritis, haemolytic uremic syndrome, diabetic nephropathy or hypertensive nephrosclerosis; various imflammatory diseases, such as arthritis, especially rheumatoid arthritis, inflammatory bowel disease, psorsasis, sarcoidosis, arterial arteriosclerosis and diseases occurring after transplants, endometriosis or chronic asthma and other conditions; disease states including, e.g., edema associated with tumors including, e.g., brain tumors; ascites associated with malignancies; Meigs' syndrome; lung inflammation; nephrotic syndrome; pericardial effusion; pleural effusion,; permeability associated with cardiovascular diseases such as the condition following myocardial infarctions and strokes and the like.

Examples of cancer to be treated herein include, but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL: high grade lymphoblastic NHL; high grade small non-cleaved cell NHL; bulky disease NHL: mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome. More particularly, cancers that are amenable to treatment by the antibodies of the invention include breast cancer, colorectal cancer, rectal cancer, non-small cell lung cancer, non-Hodgkins lymphoma (NHL), renal cell cancer, prostate cancer, liver cancer, pancreatic cancer, soft-tissue sarcoma, Kaposi's sarcoma, carcinoid carcinoma, head and neck cancer, melanoma, ovarian cancer, mesothelioma, and multiple myeloma.

It is contemplated that when used to treat various diseases such as tumors, the VEGF antagonist can be combined with one or more other therapeutic agents suitable for the same or similar diseases. For example, when used for treating cancer, the VEGF antagonist may be used in combination with conventional anti-cancer therapies, such as surgery, radiotherapy, chemotherapy or combinations thereof

In certain embodiments, the VEGF antagonist (e.g., anti-VEGF antibody) and the one or more other therapeutic agents can be administered simultaneously or sequentially in an amount and for a time sufficient to treat tumors. In certain embodiments, the VEGF antagonist can be administered subsequent to other anti-cancer agents. In certain embodiments, the VEGF antagonist is administered simultaneously with cancer therapy, e.g., chemotherapy. Alternatively, or additionally, the antagonist therapy alternates with another cancer therapy, which can be performed in any order.

In certain aspects, other therapeutic agents useful for combination cancer therapy with the VEGF antagonist include other anti-angiogenic agents. Many anti-angiogenic agents have been identified and are known in the arts, including those listed by Carmeliet and Jain (2000) Nature 407(6801):249-57 and those described herein under Definitions and Angiogenic Inhibitors sections.

In certain embodiments, the VEGF antagonist maybe used in combination with bFGF antagonist. In certain embodiment, the VEGF antagonist is an anti-VEGF antibody. In certain embodiments, the bFGF antagonist is an anti-bFGF antibody. In certain embodiment, anti-VEGF antibody bevacizumab maybe used in combination with bFGF antagonist. In certain embodiment, bevacizumab maybe used in combination with anti-bFGF antibody. In certain embodiments, the anti-VEGF antibody and anti-bFGF antibody can be administered simultaneously or sequentially in an amount and for a time sufficient to treat tumors.

The agents of the disclosure (*e.g.*, VEGF antagonist, bFGF antagonist, chemotherapeutic agent, or anti-cancer agent) are administered to a human patient, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes, and/or subcutaneous administration.

Where the method of the disclosure contemplates administration of an antibody to a patient, depending on the type and severity of the disease, about 1 µg/kg to 50 mg/kg *(e.g.* 0.1-20mg/kg) of antibody is an initial candidate dosage for administration to the patient, whether, for example, by one or more separate administrations, or by continuous infusion. A typical daily dosage might range from about 1 µg/kg to about 100 mg/kg or more, depending on the factors mentioned above. For repeated administrations over several days or longer, depending on the condition, the treatment is sustained until a desired suppression of disease symptoms occurs. However, other dosage regimens may be useful. In certain embodiments, the antibody is administered every two to three weeks, at a dose ranged from about 5 mg/kg to about 15 mg/kg. In one aspect, the antibody is administered every two to three weeks at a dose of about 5 mg/kg, 7.5 mg/kg, 10 mg/kg or 15 mg/kg. Such dosing regimen may be used in combination with a chemotherapy regimen. In some aspects, the chemotherapy regimen involves the traditional high-dose intermittent administration. In certain embodiments, the chemotherapeutic agents are administered using smaller and more frequent doses without scheduled breaks ("metronomic chemotherapy"). The progress of the therapy is easily monitored by conventional techniques and assays.

The effective amounts of therapeutic agents, e.g., bFGF antagonist, administered in combination with a VEGF antagonist will be at the physicians's discretion. Suitable dosages for the VEGF antagonist are those presently used and can be lowered due to the combined action (synergy) of the VEGF antagonist and the different antagonist, e.g., bFGF antagonist.

The antibody composition will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the antibody to be administered will be governed by such considerations, and is the minimum amount necessary to prevent, ameliorate, or treat a disease or disorder. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as used hereinbefore or about from 1 to 99% of the heretofore employed dosages. Generally, alleviation or treatment of a disease or disorder involves the lessening of one or more symptoms or medical problems associated with the disease or disorder. In the case of cancer, the therapeutically effective amount of the drug can accomplish one or a combination of the following: reduce the number of cancer cells; reduce the tumor size; inhibit *(i.e.,* to decrease to some extent and/or stop) cancer cell infiltration into peripheral organs; inhibit tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. In some embodiments, a composition of this disclosure can be used to prevent the onset or reoccurrence of the disease or disorder in a subject or mammal.

In certain aspects, the disclosure provides a method of identifying a patient with a cancer who may benefit from anti-angiogenic therapy comprising determining expression levels of bFGF in a sample obtained from the patient and then treating the patient by administering effective amounts of a VEGF antagonist and one or more chemotherapeutic agents to the patient. A variety of chemotherapeutic agents may be used in the combined treatment methods of the invention. An exemplary and non-limiting list of chemotherapeutic agents contemplated is provided herein in the section providing definitions of the terms used in this specification.

In certain embodiments, the methods of the present disclosure comprise administering to the patient a VEGF antagonist and an effective amount of at least one chemotherapeutic agent. In certain embodiments, the chemotherapeutic agent is cisplatin. In certain embodiments, the chemotherapeutic agent is gemcitabine. In certain embodiments, the chemotherapeutic agent is carboplatin. In certain embodiments, the chemotherapeutic agent is paclitaxel. In certain embodiments, effective amounts of VEGF antagonist, cisplatin and gemcitabine are administered to the patient. In certain embodiments, effective amounts of VEGF antagonist, carboplatin and paclitaxel are administered to the patient.

As will be understood by those of ordinary skill in the art, the appropriate doses of chemotherapeutic agents will be generally around those already employed in clinical therapies wherein the chemotherapeutics are administered alone or in combination with other chemotherapeutics. Variation in dosage will likely occur depending on the condition being treated. The physician administering treatment will be able to determine the appropriate dose for the individual subject.

### EXAMPLES

### Example 1: AVAiL Trial Protocol

Addition of bevacizumab to platinum-based chemotherapy in 2 phase III trials, E4599 and AVAiL, improved outcome in patients with untreated advanced NSCLC. In study E4599, it was an objective of the study to determine the prognostic and predictive value of several biomarkers. This analysis of several biomarkers showed that intracellular adhesion molecule-1 (ICAM-1) levels may be predictive of response to therapy and prognostic of survival, whereas patients with high baseline vascular endothelial growth factor (VEGF) levels may have a higher response rate to bevacizumab. In AVAiL, a similar biomarker analysis was performed.

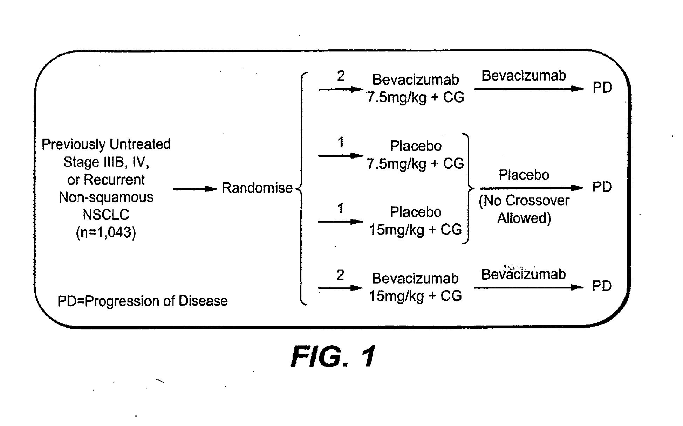

The AVAiL ("AVASTIN™ in Lung", BO177704) study is a randomized, controlled, double-blind phase III study that included more than 1,000 patients with previously untreated advanced non-small cell lung cancer (NSCLC) with histology other than squamous cell (Figure 1). The primary objective of the study was to demonstrate superiority in patient progression free survival (PFS) of both AVASTIN™ containing treatment arms versus the control regimen. Secondary endpoints of the study included overall survival (OS), response rate and duration of response, quality of life and a safety comparison of the two doses of AVASTIN™ versus standard of care. Two doses of AVASTIN™ were studied (7.5 mg/kg and 15 mg/kg). The AVAiL study confirmed the clinical benefit in terms of significantly increased PFS (PFS rates that were 20 to 30% higher than those who received chemotherapy alone) already found in the NSCLC (E4599) trial (Reck M, et al. J Clin Oncol 2009; 27(8):1227-1234; Sandler A.B. et al.; N Engl. J. Med. 2006; 355: 2542-2550). A similar treatment effect was observed both at a dosage of 7.5 and 15 mg/kg AVASTIN™.

The trial protocol was approved by the institutional review board at each site and was conducted in accordance with the Declaration of Helsinki, current US Food and Drug Administration Good Clinical Practices, and local ethical and legal requirements. The decision to perform retrospective biomarker analyses for all patients randomized to the AVAiL trial was taken after recruitment to the study was completed.

Complete demographic profile of the set of patients was taken and the full medical history of each patient was taken on record. All patients underwent a physical examination including weight, height vital signs and cardiopulmonary examination, ECOG performance status, 12-lead ECG. The tumor status of each patient was assessed as described previously (Reck M. et al., J. Clin. Oncol. 2009; 27(9): 1227-1234). Briefly, tumor status was assessed every three cycles (approximately every 9 weeks) during trial treatment. If patients withdrew from trial treatment for reasons other than progressive disease, follow-up tumor assessments were repeated every two months until disease progression. Tumor responses were assessed by the investigator according to Response Evaluation Criteria in Solid Tumors (Therasse P. et al., J. Clin. Oncol. 2009; 27(9):1227-1234). An independent Data and Safety Monitoring Board was responsible for ongoing review of unblended safety data. Adverse events (AE) were graded using the National Cancer Institute Common Toxicity Criteria for Adverse Events (version 3.0) and coded according to the medical dictionary for regulatory activities. Laboratory data were also obtained and provided to the Data and Safety Monitoring Board.

The biomarker population baseline characteristics for patients receiving low-dose bevacizumab (7.5 mg/kg) are shown in Table 1 below. Baseline demographic characteristics for patients included in the biomarker analysis shown in Table 1 were reflective of the study population as a whole.

**Table 1**

| | Placebo+cis/gem N = 112 | Bv7.5+cis/gem N = 131 |
|---|---|---|
| Sex | | |
| FEMALE | 40 (36%) | 48 (37%) |
| MALE | 72 (64%) | 83 (63%) |
| n | 112 | 131 |
| | | |

| Smoking Status | | |
|---|---|---|
| NEVER SMOKED | 26 (23%) | 33 (25%) |
| PAST SMOKER | 58 (52%) | 57 (44%) |
| CURRENT SMOKER | 28 (25%) | 41 (31%) |
| n | 112 | 131 |
| | | |

| Age Category Years | | |
|---|---|---|
| <65 | 76 (68%) | 97 (74%) |
| >=65 | 36 (32%) | 34 (26%) |
| n | 112 | 131 |

| Disease Stage | | |
|---|---|---|
| IIIB (NOT RECURRENT) | 14 (13%) | 20 (15%) |
| IV (NOT RECURRENT) | 90 (80%) | 98 (75%) |
| RECURRENT | 8 (7%) | 13 (10%) |
| n | 112 | 131 |
| | | |

| Region Category | | |
|---|---|---|
| CENTRAL AND SOUTH | 1 (<1%) | - |
| AMERICA | | |
| EAST ASIA | 3 (3%) | 11 (8%) |
| EASTERN EUROPE | 28 (25%) | 31 (24%) |
| WEST EUROPE/ AUSTRALIA/ CANADA | 80 (71%) | 89 (68%) |
| n | 112 | 131 |
| | | |

| Race | | |
|---|---|---|
| Asian | 5 (4%) | 13 (10%) |
| White | 107 (96%) | 117 (89%) |
| Other | - | 1 (<1%) |
| n | 112 | 131 |
| | | |

| ECOG (Baseline) | | |
|---|---|---|
| | | |
| 0 | 45 (40%) | 52 (40%) |
| 1 | 67 (60%) | 79 (60%) |
| n | 112 | 131 |
| | | |

| Cellular Classification | | |
|---|---|---|
| ADENOCARCINOMA | 96 (86%) | 116 (89%) |
| LARGE CELL CARCINOMA | 13 (12%) | 8 (6%) |
| MIXED PREDOMINANTLY ADENOCARCINOMA | - | 1 (<1%) |
| OTHER | 3 (3%) | 6 (5%) |
| n | 112 | 131 |
| | | |

| Baseline LDH | | |
|---|---|---|
| <= ULN | 62 (58%) | 82 (67%) |
| > ULN | 45 (42%) | 40 (33%) |
| n | 107 | 122 |
| | | |

| Baseline Platelets | | |
|---|---|---|
| <=300 10*9/L | 49 (44%) | 69 (53%) |
| >300 10*9/L | 63 (56%) | 61 (47%) |
| n | 112 | 130 |

### Example 2: Sample Collection and Analysis

Plasma samples were collected from patients with stage IIIB or IV or recurrent non-squamous NSCLC patients before any drug was administered. All samples were obtained from patients that hereafter were treated with cisplatin 80 mg/m² and gemcitabine 1,250 mg/m² for up to six cycles plus low-dose bevacizumab (7.5 mg/kg), high-dose bevacizumab (15 mg/kg), or placebo every 3 weeks until disease progression.

A total of 3 mLs of blood were drawn into a sodium citrate vaccutainer tube. They were mixed immediately by invertion of the tube 10 to 15 times and were centrifuged at approximately 3000g in a 4°C refrigerated centrifuge. Plasma was aliquoted into the 2 plastic vials. Samples were stored in an upright position at -70°C. In some cases, samples were stored at -20°C for up to one month and then transferred to -70°C.

All samples were thawed and distributed in 40 µl aliquots into 384-well REMP micro tube plates in batches of 72. The tubes were sealed with an aluminum septum and stored in freezers set to maintain a temperature of -70°C until analysis. A total of 366 samples were available for analysis of bFGF concentrations.

Sandwich immunoassay for measuring bFGF was purchased from R&D Systems (Minneapolis, MN). The kits were used according to the manufacturer's recommendations. Briefly, a basic standard was prepared with Calibrator Diluent at a stock solution of 64 pg/mL. The standard dilution series in Calibrator Diluent included 32, 16, 8, 4, 2, 1 and 0 pg/mL bFGF.

Standard, control or samples prediluted in assay buffer were added into each well of a polystyrene microplates coated with a mouse monoclonal antibody against bFGF as provided in the assay kit. After 3 hour incubation at room temperature, the plates were washed, and conjugated detection antibody was added and incubated for additional 2 hours. Following another wash, 50 µl substrate solution was added in each well, and incubated for 45 minutes at room temperature. Hereafter, amplifier solution (50 µl/well) was added, color was allowed to develop for 45 minutes, and the reaction was stopped by 2N sulfuric Acid (50 µl/well). Within 30 minutes, the plates were read at a wave length of 490 nm using a Microplate Spectrophotometer (Versamax, Molecular Devices, Sunnyvale, CA). Absorbance values at 490 nm were corrected for the absorbance at 650 nm. Data reduction was based on 4 parameter logistic curve fit, using the instruments software package. The bFGF protein level was measured in pg/ml, ranging from 2-64 pg/ml due to the functional limits of the assay.

In case the bFGF protein levels were outside the range of detection, results were reported as BLQ (Below Limit of Quantification) or ALQ (Above the upper Limit of Quantification). As sample volume was limited for bFGF measurements, only single measurements were performed. If sample volume was not sufficient for measurements, even for a single replicate measurement, QNS (quantity not sufficient) was reported. If a single measurement was possible, the result from the single measurement was reported.

Based on the three in-run control samples that were included on each test plate, performance of the bFGF assay during sample analysis was satisfactory throughout the conduct of the study. The numbers of samples yielding valid results, results below or above the quantification range, or no valid results are summarized in Table 2 below.

**Table 2**

| **Assay Molecule** | **Result within range (n)** | **ALQ** | **BLQ** * | **NOA** | **QNS** |
|---|---|---|---|---|---|
| bFGF | 249 | 22 | 52 | 6 | 43 |

### Example 3: Statistical Analysis of bFGF Protein Level Data

For statistical analysis, the data relating to bFGF protein expression levels were divided in two categories: high level and low level. The definition of high and low level is provided herein.

Overall survival (OS) end-point was defined as the time from entry in the study until death or censoring. The progression free survival (PFS) end point was defined as time from entry in the study until disease progression or death.

The cox proportional hazard regression model was used to evaluate the effect of predictor variables on PFS and OS. The hazard ratio for OS was defined as the ratio of the risk of death in treated group divided by risk of death in placebo group. A hazard ratio below 1 indicates that the risk of death is less in the treated arm than in the placebo arm, and therefore that the mean OS is longer in treated patients than in placebo patients. The hazard ratio for PFS was defined as the ratio of the risk of death or progression in treated group divided by risk of death in placebo group. A hazard ratio below 1 indicates that the risk of progression or death is less in the treated arm than in the placebo arm, and therefore that the mean PFS is longer in treated patients than in placebo patients.

"Treatment effect" or "effect of treatment" was defined as the effect on OS or PFS that was observed when 7.5 mg/kg of bevacizumab was administered to the patients compared to placebo. Treatment effect is assessed using the hazard ratio. A positive treatment was defined to mean that the OS or PFS was increased in treated arm compared to placebo arm. Therefore, a positive treatment effects corresponds to a hazard ratio below 1.

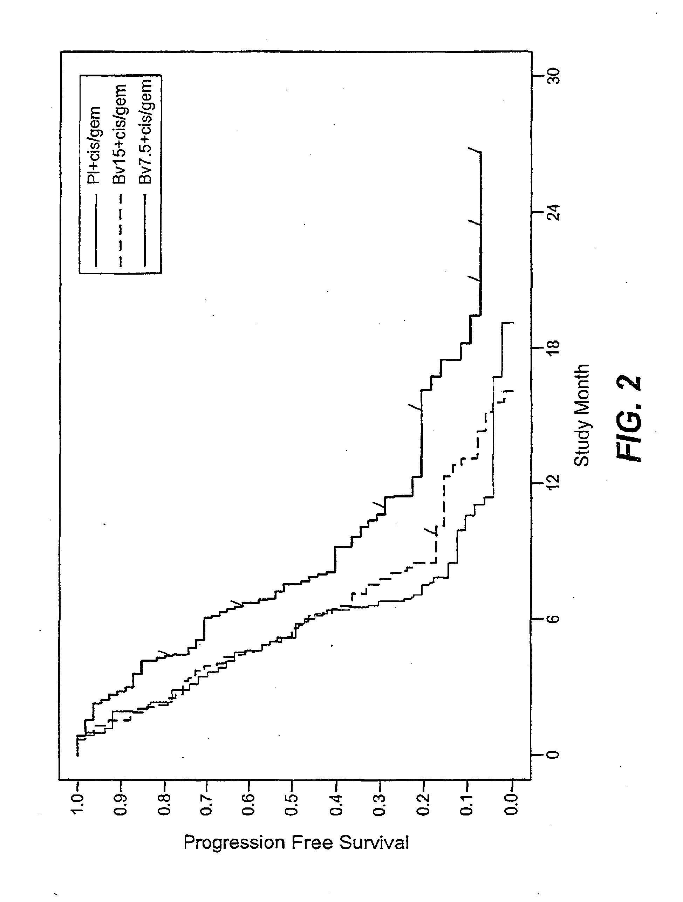

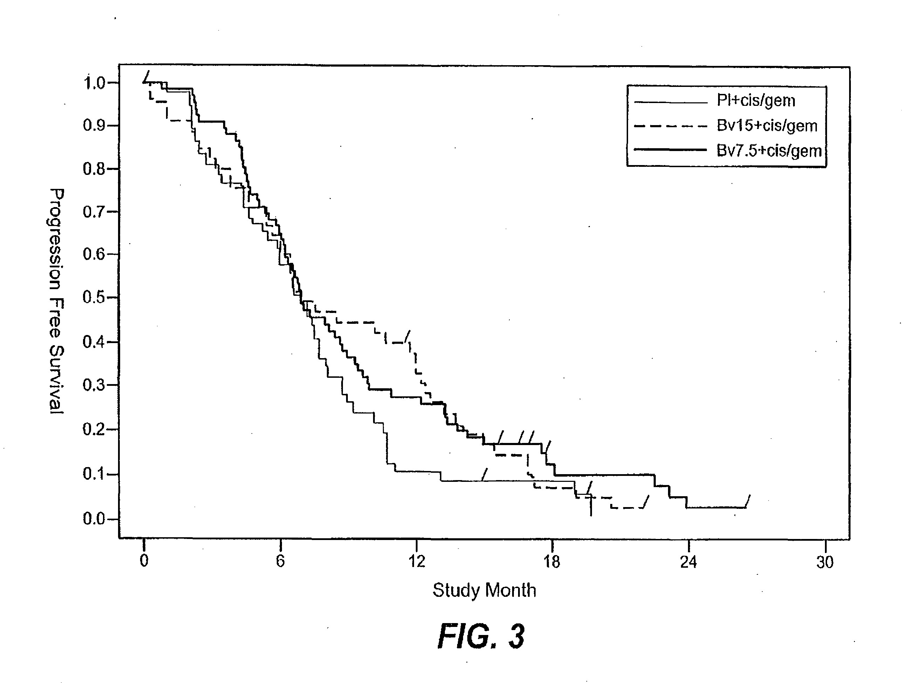

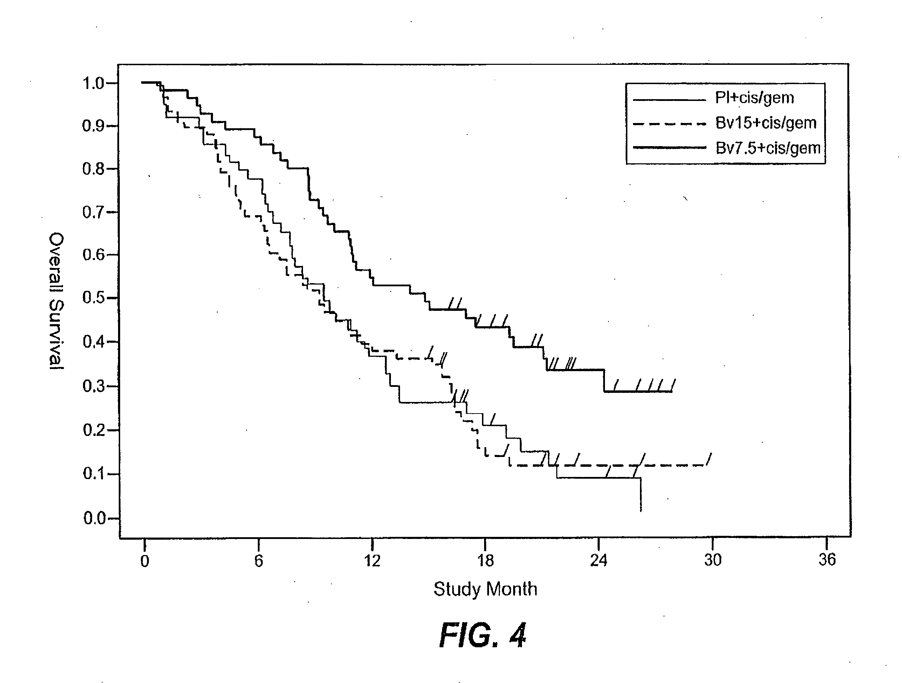

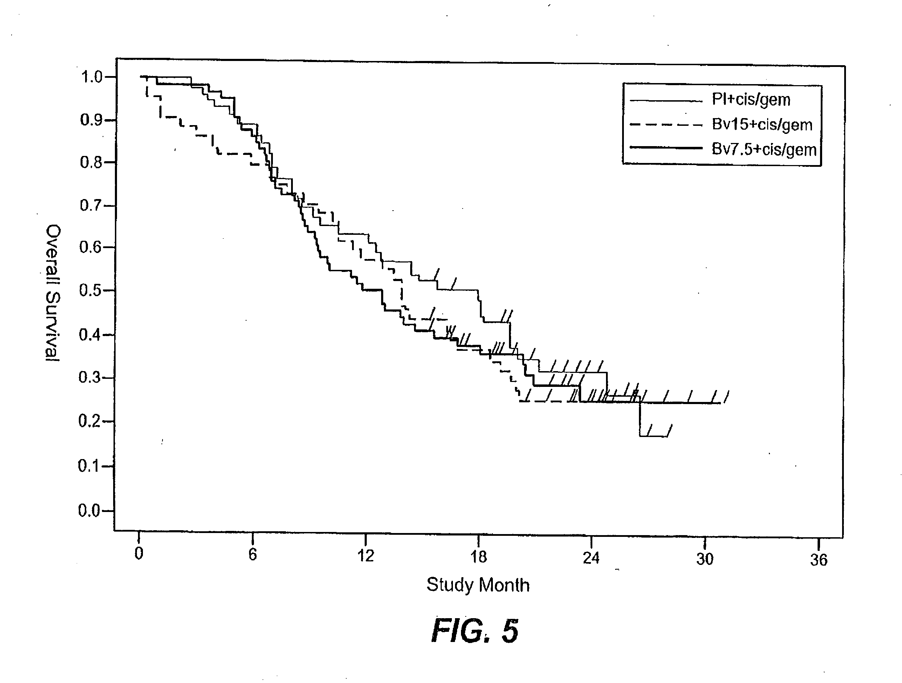

The Kaplan Meier method was used to generate graphical display of survival probability by treatment group in sub-group of patients with high bFGF protein level and subgroup of patients with low bFGF protein level. (Figures 2 to 5).

The first set of analysis evaluated the treatment effect of patients with high bFGF protein level. The median value of bFGF level from the samples was 6.9 pg/ml. Therefore, in the first example, if the bFGF protein level in a sample obtained from a patient was greater than 6.9 pg/ml, then the sample was designated as having a high level of bFGF protein. Additionally, cutoff value of 2 pg/ml was used to further characterize the effect of the biomarker since the value corresponded to the lower limit of assay detection. Therefore, in the second example, if the bFGF protein level in a sample obtained from a patient was greater than 2 pg/ml, then the sample was designated as having a high level of bFGF protein.

For OS, the null hypothesis stated that there is no difference in OS between the placebo treatment group and 7.5 mg/kg bevacizumab treatment group. The alternative hypothesis stated that there is a difference in OS between the two treatment groups. A log-rank test was used to calculate the p-value corresponding to this hypothesis. P-value below 0.05 is considered to be statistically significant.

For PFS the null hypothesis stated that there is no difference in PFS between the placebo treatment group and 7.5 mg/kg bevacizumab treatment group. The alternative hypothesis stated that there is a difference in PFS between the two treatment groups. A log-rank test was used to calculate the p-value corresponding to this hypothesis. P-value below 0.05 is considered to be statistically significant.

The second set of analysis evaluated the treatment effect in patients with low bFGF protein level. In the first example, if the bFGF protein level in a sample obtained from a patient was equal or less than 6.9 pg/ml, then the sample was designated as having a low level of bFGF protein. In the second example, if the bFGF protein level in a sample obtained from a patient was equal or less than 2 pg/ml, then the sample was designated as having a low level of bFGF protein.

The null hypothesis stated that there is no difference in OS between the two treatment groups. The alternative hypothesis stated that there is a difference in OS between the treatment groups. A log-rank test was used to calculate the p-value corresponding to this hypothesis. P-value below 0.05 is considered to be statistically significant.

For PFS the null hypothesis stated that there is no difference in Progression Free Survival between the two treatment groups. The alternative hypothesis stated that there is a difference in PFS between the treatment groups. A log-rank test was used to calculate the p-value corresponding to this hypothesis. P-value below 0.05 is considered to be statistically significant.

The third set of analysis was conducted in order to evaluate if treatment effect differed according to bFGF protein level. A model with treatment group, bFGF level and treatment by bFGF interaction as predictor was used.

For OS, the null hypothesis for this interaction test stated that there is no difference in treatment effect on OS according to bFGF level. The alternative hypothesis stated that treatment effect differed according to bFGF level. A likelihood ratio test was used to calculate the p-value corresponding to this hypothesis. P-value below 0.05 is considered to be statistically significant. P-value below 0.1 is considered borderline significant.

For PFS, the null hypothesis for this interaction test stated that there is no difference in treatment effect on PFS according to bFGF level. The alternative hypothesis stated that treatment effect differed according to bFGF level. P-value below 0.05 is considered to be statistically significant. P-value below 0.1 is considered borderline significant.

A fourth set of analysis was conducted in order to check the robustness of results found with the other three analyses described above. In this set of analysis, the values for bFGF protein levels were not dichotomized but treated as continuous variable after a logarithmic transformation. In addition, other baseline prognostic variables were included in the model in order to check whether the effect of bFGF was not confounded by other factors. The model had treatment group, bFGF level, treatment by bFGF interaction and other baseline covariates as predictors. The other baseline predictors were: gender (male vs. female); past smoker vs. all other; age (<65 vs. >=65); disease stage (IV vs. all other); West Europe/ Australia/ Canada vs. all other; race (white vs. all other); ECOG at screening; adenocarcinoma vs. all other.

For Overall Survival, the null hypothesis stated that the treatment effect on OS did not vary in a linear fashion with varying bFGF protein expression levels. The alternative hypothesis stated that treatment effect varied with varying bFGF protein expression levels. A likelihood ratio test was used to calculate the p-value corresponding to this hypothesis. P-value below 0.05 is considered to be statistically significant.

For PFS, the null hypothesis stated that there treatment effect on PFS did not vary in a linear fashion with varying bFGF protein expression levels. The alternative hypothesis stated that treatment effect varied with varying bFGF protein expression levels. A likelihood ratio test was used to calculate the p-value corresponding to this hypothesis. P-value below 0.05 is considered to be statistically significant.

Table 3 below shows the results of analyses set 1 and 2 evaluating the effect of treatment on in sub-groups of patients with low protein expression level of bFGF defined as values ≤ 6.9 pg/ml and high protein expression level of bFGF defined as values > 6.9 pg/ml.

Results in Table 3 show a strong treatment effect on PFS and OS in patients with high protein expression level of bFGF (> 6.9 pg/ml). Hazard ratio estimates well below 1 and p-value below 0.05 both indicate that treatment significantly prolongs time to death and time to progression in patients with bFGF protein level of > 6.9 pg/ml.

**Table 3**

| | Bevacizumab 7.5mg/kg vs placebo | | | |
|---|---|---|---|---|
| Analysis 1 and 2 | Progression Free Survival | | Overall Survival | |
| | HR [95%CI]* | p-value | HR [95%CI] | p-value |
| Low bFGF Protein Level (≤ 6.9 pg/ml) | 0.74 [0.50;1.09] | 0.12 | 1.13 [0.72;1.76] | 0.5979 |
| High bFGF Protein Level (> 6.9 pg/ml) | 0.47 [0.31;0.71] | 0.0002 | 0.52 [0.33;0.81] | 0.0036 |

| | | | | |
|---|---|---|---|---|
| * HR [95%CI]: Hazard Ratio with 95% confidence interval. | | | | |

Table 4 below shows the results of analysis set 1 and 2 evaluating the effect of treatment on PFS and OS in sub-groups of patients with low protein expression level of bFGF defined as values ≤ 2 pg/ml and high protein expression level of bFGF defined as values > 2pg/ml.

Results in Table 4 show a strong treatment effect on PFS and OS in patients with high protein expression level of bFGF (> 2 pg/ml). Hazard ratio estimates well below 1 and p-value below or at the 0.05 level both indicate that treatment significantly prolongs time to death and time to progression in patients with bFGF protein level of >2pg/ml.

**Table 4**

| | Bevacizumab 7.5 mg/kg vs placebo | | | |
|---|---|---|---|---|
| Analysis 1 and 2 | Progression Free Survival | | Overall Survival | |
| | HR [95%CI]* | p-value | HR [95%CI] | p-value |
| Low bFGF Protein Level (≤ 2pg/ml) | 0.73 [0.37 -1.47] | 0.38 | 1.29 [0.56 -3.0] | 0.553 |
| High bFGF Protein Level (> 2 pg/ml) | 0.56 [0.41 0.77] | 0.0003 | 0.71 [0.51- 1] | 0.0506 |

| | | | | |
|---|---|---|---|---|
| * HR [95%CI] : Hazard Ratio with 95% confidence interval. | | | | |

Table 5 below shows the result of the third set of analysis. It reports the p-values for test of interaction between treatment effect and bFGF protein expression level. These results provide additional evidence that the treatment effect is significantly more important in the sub-group of patients with high protein expression level of bFGF.

**Table 5**

| Analysis 3 | Bevacizumab 7.5 mg/kg vs placeboby (≤ 6.9 pg/ml) vs (> 6.9 pg/ml bFGF) |
|---|---|
| | p-value |
| Progression Free Survival | 0.0807 |
| Overall Survival | 0.0126 |

Table 6 below shows the outcome of the fourth set of analysis. It displays the p-value for the treatment by interaction term, in a model using values for bFGF protein expression level as logarithmic transformed continuous variables and other baseline patient characteristics. These results provide additional evidence that the treatment effect is significantly associated with bFGF protein expression level, wherein the higher treatment effect was observed in patients having higher protein expression levels of bFGF.

**Table 6**

| Analysis 4 | [Bevacizumab 7.5mg/kg vs placebo] by (≤ 6.9 ng/ml) vs (> 6.9 ng/ml bFGF) |
|---|---|
| | p-value |
| Progression Free Survival | 0.0339 |
| Overall Survival | 0.0446 |

In summary, several statistical models were applied to assess the treatment effect on PFS and OS in relation to the bFGF level. Analyses were first performed with high protein level bFGF defined as all bFGF protein expression levels with values > 2 pg/ml. Next, in order to increase the statistical power to detect a difference between high and low bFGF protein expression levels in terms of treatment effect, analyses were also performed with high protein expression level bFGF defined as all values > 6.9 pg/ml. All analyses consistently indicated that a significant increase in PFS and OS was present in patients with high protein expression level bFGF (Figures 2, 3,4 and 5).

While the present invention has been described with reference to what are considered to be the specific embodiments, it is to be understood that the invention is not limited to such embodiments.

Throughout the present application, including the claims, the term "comprising" is used as an inclusive, open-ended transition phrase, which does not exclude additional, unrecited elements or method steps.

## Claims

1. A method of identifying a patient with a lung cancer who may benefit from anti-angiogenic therapy comprising an anti-VEGF antibody and at least one chemotherapeutic agent, said method comprising determining expression level of bFGF in a blood sample obtained from the patient, wherein higher expression level of bFGF in the sample obtained from the patient as compared to a reference sample indicates that the patient may benefit from anti-angiogenic therapy.

2. A method of predicting responsiveness of a patient with a lung cancer to anti-angiogenic therapy comprising an anti-VEGF antibody and at least one chemotherapeutic agent, said method comprising determining expression level of bFGF in a blood sample obtained from the patient, wherein higher expression level of bFGF in the sample as compared to a reference sample indicates that the patient is likely to be responsive to the anti-angiogenic therapy.

3. The method of claim 1 or claim 2, wherein the anti-VEGF antibody is bevacizumab.

4. The method of claim 3, wherein the chemotherapeutic agent is cisplatin or gemcitabine.

5. The method of claim 3, wherein the anti-angiogenic therapy comprises at least two chemotherapeutic agents.

6. The method of claim 5, wherein at least two chemotherapeutic agents are cisplatin and gemcitabine.

7. The method of any one of claims 1 to 6, wherein the anti-angiogenic therapy comprises 7.5 mg/kg of bevacizumab.

8. The method of any one of claims 1 to 7, wherein the expression level of bFGF is a protein expression level.

9. The method of any one of claims 1 to 7, wherein the protein expression level of bFGF in the sample is greater than 2 pg/ml.

10. The method of any one of claims 1 to 7, wherein the protein expression level bFGF in the sample is greater than 6.9 pg/ml.

11. The method of any one of claims 1 to 10, wherein the cancer is previously untreated lung cancer.

12. The method of any one of claims 1 to 10, wherein the cancer is non-small cell lung cancer.

13. The method of any one of claims 1 to 7, wherein the sample obtained from the patient is a plasma sample.

14. Use of a protein or an oligonucleotide or a polynucleotide array in a method of any one of claim 1 to 7, wherein the protein or the oligonucleotide or the polynucleotide array is used for determining the expression level of bFGF.

## Patentansprüche

1. Verfahren zur Identifizierung eines Patienten mit einem Lungenkrebs, der von Antiangiogenese-Therapie profitieren könnte, die einen anti-VEGF-Antikörper und mindestens ein chemotherapeutisches Mittel umfasst, wobei das Verfahren die Bestimmung des Expressionsspiegels von bFGF in einer vom Patienten erhaltenen Blutprobe umfasst, wobei ein höherer Expressionsspiegel von bFGF in der vom Patienten erhaltenen Probe, verglichen mit einer Referenzprobe, anzeigt, dass der Patient von Antiangiogenese-Therapie profitieren könnte.

2. Verfahren zur Vorhersage, ob ein Patient mit einem Lungenkrebs auf Antiangiogenese-Therapie anspricht, die einen anti-VEGF-Antikörper und mindestens ein chemotherapeutisches Mittel umfasst, wobei das Verfahren die Bestimmung des Expressionsspiegels von bFGF in einer vom Patienten erhaltenen Blutprobe umfasst, wobei ein höherer Expressionsspiegel von bFGF in der vom Patienten erhaltenen Probe, verglichen mit einer Referenzprobe, anzeigt, dass der Patient wahrscheinlich auf die Antiangiogenese-Therapie ansprechen wird.

3. Verfahren nach Anspruch 1 oder Anspruch 2, wobei der anti-VEGF-Antikörper Bevacizumab ist.

4. Verfahren nach Anspruch 3, wobei das chemotherapeutische Mittel Cisplatin oder Gemcitabin ist.

5. Verfahren nach Anspruch 3, wobei die Antiangiogenese-Therapie mindestens zwei chemotherapeutische Mittel umfasst.

6. Verfahren nach Anspruch 5, wobei die mindestens zwei chemotherapeutischen Mittel Cisplatin und Gemcitabin sind.

7. Verfahren nach einem der Ansprüche 1 bis 6, wobei die Antiangiogenese-Therapie 7,5 mg/kg Bevacizumab umfasst.

8. Verfahren nach einem der Ansprüche 1 bis 7, wobei der Expressionsspiegel von bFGF ein Proteinexpressionsspiegel ist.

9. Verfahren nach einem der Ansprüche 1 bis 7, wobei der Proteinexpressionsspiegel von bFGF in der Probe größer als 2 pg/ml ist.

10. Verfahren nach einem der Ansprüche 1 bis 7, wobei der Proteinexpressionsspiegel von bFGF in der Probe größer als 6,9 pg/ml ist.

11. Verfahren nach einem der Ansprüche 1 bis 10, wobei der Krebs zuvor unbehandelter Lungenkrebs ist.

12. Verfahren nach einem der Ansprüche 1 bis 10, wobei der Krebs nichtkleinzelliger Lungenkrebs ist.

13. Verfahren nach einem der Ansprüche 1 bis 7, wobei die vom Patienten erhaltene Probe eine Plasmaprobe ist.

14. Verwendung eines Protein- oder eines Oligonucleotid- oder eines Polynucleotidarrays in einem Verfahren nach einem der Ansprüche 1 bis 7, wobei der Protein- oder der Oligonucleotid- oder der Polynucleotidarray zur Bestimmung des Expressionsspiegels von bFGF verwendet wird.

## Revendications

1. Procédé d'identification d'un patient ayant un cancer du poumon qui peut bénéficier d'une thérapie anti-angiogène comprenant un anticorps anti-VEGF et au moins un agent chimiothérapeutique, ledit procédé comprenant la détermination du niveau d'expression de bFGF dans un échantillon sanguin obtenu auprès du patient, où un plus haut niveau d'expression de bFGF dans l'échantillon obtenu auprès du patient que dans un échantillon de référence indique que le patient peut bénéficier d'une thérapie anti-angiogène.

2. Procédé de prédiction de la sensibilité d'un patient ayant un cancer du poumon à une thérapie anti-angiogène comprenant un anticorps anti-VEGF et au moins un agent chimiothérapeutique, ledit procédé comprenant la détermination du niveau d'expression de bFGF dans un échantillon sanguin obtenu auprès du patient, où un plus haut niveau d'expression de bFGF dans l'échantillon que dans un échantillon de référence indique que le patient est susceptible d'être sensible à la thérapie anti-angiogène.

3. Procédé selon la revendication 1 ou la revendication 2 où l'anticorps anti-VEGF est le bévacizumab.

4. Procédé selon la revendication 3 où l'agent chimiothérapeutique est le cisplatine ou la gemcitabine.

5. Procédé selon la revendication 3 où la thérapie anti-angiogène comprend au moins deux agents chimiothérapeutiques.

6. Procédé selon la revendication 5 où au moins deux agents chimiothérapeutiques sont le cisplatine et la gemcitabine.

7. Procédé selon l'une quelconque des revendications 1 à 6 où la thérapie anti-angiogène comprend 7,5 mg/kg de bévacizumab.

8. Procédé selon l'une quelconque des revendications 1 à 7 où le niveau d'expression de bFGF est un niveau d'expression de protéine.

9. Procédé selon l'une quelconque des revendications 1 à 7 où le niveau d'expression de protéine de bFGF dans l'échantillon est supérieur à 2 pg/ml.

10. Procédé selon l'une quelconque des revendications 1 à 7 où le niveau d'expression de protéine de bFGF dans l'échantillon est supérieur à 6,9 pg/ml.

11. Procédé selon l'une quelconque des revendications 1 à 10 où le cancer est un cancer du poumon non traité antérieurement.

12. Procédé selon l'une quelconque des revendications 1 à 10 où le cancer est un cancer du poumon non à petites cellules.

13. Procédé selon l'une quelconque des revendications 1 à 7 où l'échantillon obtenu auprès du patient est un échantillon de plasma.

14. Utilisation d'une protéine ou d'un oligonucléotide ou d'une puce à polynucléotides dans un procédé selon l'une quelconque des revendications 1 à 7 où la protéine ou l'oligonucléotide ou la puce à polynucléotides est utilisé(e) pour déterminer le niveau d'expression de bFGF.


### Sequence Listing

<110> F. Hoffmann-La Roche AG
<120> METHODS AND COMPOSITIONS FOR DIAGNOSTICS USE IN CANCER PATIENTS
<130> 26419
<141> 2009-09-17
<160> 5
<210> 1
   <211> 288
   <212> PRT
   <213> Homo sapiens
<400> 1
   Met Val Gly Val Gly Gly Gly Asp Val Glu Asp Val Thr Pro Arg
   1               5                   10                  15
   Pro Gly Gly Cys Gln Ile Ser Gly Arg Gly Ala Arg Gly Cys Asn
                   20                  25                  30
   Gly Ile Pro Gly Ala Ala Ala Trp Glu Ala Ala Leu Pro Arg Arg
                   35                  40                  45
   Arg Pro Arg Arg His Pro Ser Val Asn Pro Arg Ser Arg Ala Ala
                   50                  55                  60
   Gly Ser Pro Arg Thr Arg Gly Arg Arg Thr Glu Glu Arg Pro Ser
                   65                  70                  75
   Gly Ser Arg Leu Gly Asp Arg Gly Arg Gly Arg Ala Leu Pro Gly
                   80                  85                  90
   Gly Arg Leu Gly Gly Arg Gly Arg Gly Arg Ala Pro Glu Arg Val
                   95                  100                 105
   Gly Gly Arg Gly Arg Gly Arg Gly Thr Ala Ala Pro Arg Ala Ala
                   110                 115                 120
   Pro Ala Ala Arg Gly Ser Arg Pro Gly Pro Ala Gly Thr Met Ala
                   125                 130                 135
   Ala Gly Ser Ile Thr Thr Leu Pro Ala Leu Pro Glu Asp Gly Gly
                   140                 145                 150
   Ser Gly Ala Phe Pro Pro Gly His Phe Lys Asp Pro Lys Arg Leu
                   155                 160                 165
   Tyr Cys Lys Asn Gly Gly Phe Phe Leu Arg Ile His Pro Asp Gly
                   170                 175                 180
   Arg Val Asp Gly Val Arg Glu Lys Ser Asp Pro His Ile Lys Leu
                   185                 190                 195
   Gln Leu Gln Ala Glu Glu Arg Gly Val Val Ser Ile Lys Gly Val
                   200                 205                 210
   Cys Ala Asn Arg Tyr Leu Ala Met Lys Glu Asp Gly Arg Leu Leu
                   215                 220                 225
   Ala Ser Lys Cys Val Thr Asp Glu Cys Phe Phe Phe Glu Arg Leu
                   230                 235                 240
   Glu Ser Asn Asn Tyr Asn Thr Tyr Arg Ser Arg Lys Tyr Thr Ser
   245 250 255
   Trp Tyr Val Ala Leu Lys Arg Thr Gly Gln Tyr Lys Leu Gly Ser
   260 265 270
   Lys Thr Gly Pro Gly Gln Lys Ala Ile Leu Phe Leu Pro Met Ser
   275 280 285
   Ala Lys Ser
<210> 2
   <211> 210
   <212> PRT
   <213> Homo sapiens
<400> 2
   Met Gly Asp Arg Gly Arg Gly Arg Ala Leu Pro Gly Gly Arg Leu
   1 5 10 15
   Gly Gly Arg Gly Arg Gly Arg Ala Pro Glu Arg Val Gly Gly Arg
   20 25 30
   Gly Arg Gly Arg Gly Thr Ala Ala Pro Arg Ala Ala Pro Ala Ala
   35 40 45
   Arg Gly Ser Arg Pro Gly Pro Ala Gly Thr Met Ala Ala Gly Ser
   50 55 60
   Ile Thr Thr Leu Pro Ala Leu Pro Glu Asp Gly Gly Ser Gly Ala
   65 70 75
   Phe Pro Pro Gly His Phe Lys Asp Pro Lys Arg Leu Tyr Cys Lys
   80 85 90
   Asn Gly Gly Phe Phe Leu Arg Ile His Pro Asp Gly Arg Val Asp
   95 100 105
   Gly Val Arg Glu Lys Ser Asp Pro His Ile Lys Leu Gln Leu Gln
   110 115 120
   Ala Glu Glu Arg Gly Val Val Ser Ile Lys Gly Val Cys Ala Asn
   125 130 135
   Arg Tyr Leu Ala Met Lys Glu Asp Gly Arg Leu Leu Ala Ser Lys
   140 145 150
   Cys Val Thr Asp Glu Cys Phe Phe Phe Glu Arg Leu Glu Ser Asn
   155 160 165
   Asn Tyr Asn Thr Tyr Arg Ser Arg Lys Tyr Thr Ser Trp Tyr Val
   170 175 180
   Ala Leu Lys Arg Thr Gly Gln Tyr Lys Leu Gly Ser Lys Thr Gly
   185 190 195
   Pro Gly Gln Lys Ala Ile Leu Phe Leu Pro Met Ser Ala Lys Ser
   200 205 210
<210> 3
   <211> 146
   <212> PRT
   <213> Homo sapiens
<400> 3
   Pro Ala Leu Pro Glu Asp Gly Gly Ser Gly Ala Phe Pro Pro Gly
   1               5                   10                  15
   His Phe Lys Asp Pro Lys Arg Leu Tyr Cys Lys Asn Gly Gly Phe
                   20                  25                  30
   Phe Leu Arg Ile His Pro Asp Gly Arg Val Asp Gly Val Arg Glu
                   35                  40                  45
   Lys Ser Asp Pro His Ile Lys Leu Gln Leu Gln Ala Glu Glu Arg
                   50                  55                  60
   Gly Val Val Ser Ile Lys Gly Val Cys Ala Asn Arg Tyr Leu Ala
                   65                  70                  75
   Met Lys Glu Asp Gly Arg Leu Leu Ala Ser Lys Cys Val Thr Asp
                   80                  85                  90
   Glu Cys Phe Phe Phe Glu Arg Leu Glu Ser Asn Asn Tyr Asn Thr
                   95                  100                 105
   Tyr Arg Ser Arg Lys Tyr Thr Ser Trp Tyr Val Ala Leu Lys Arg
                   110                 115                 120
   Thr Gly Gln Tyr Lys Leu Gly Ser Lys Thr Gly Pro Gly Gln Lys
                   125                 130                 135
   Ala Ile Leu Phe Leu Pro Met Ser Ala Lys Ser
                   140                 145
<210> 4
   <211> 135
   <212> PRT
   <213> Homo sapiens
<400> 4
   Phe Pro Pro Gly His Phe Lys Asp Pro Lys Arg Leu Tyr Cys Lys
   1               5                   10                  15
   Asn Gly Gly Phe Phe Leu Arg Ile His Pro Asp Gly Arg Val Asp
                   20                  25                  30
   Gly Val Arg Glu Lys Ser Asp Pro His Ile Lys Leu Gln Leu Gln
                   35                  40                  45
   Ala Glu Glu Arg Gly Val Val Ser Ile Lys Gly Val Cys Ala Asn
                   50                  55                  60
   Arg Tyr Leu Ala Met Lys Glu Asp Gly Arg Leu Leu Ala Ser Lys
                   65                  70                  75
   Cys Val Thr Asp Glu Cys Phe Phe Phe Glu Arg Leu Glu Ser Asn
                   80                  85                  90
   Asn Tyr Asn Thr Tyr Arg Ser Arg Lys Tyr Thr Ser Trp Tyr Val
                   95                  100                 105
   Ala Leu Lys Arg Thr Gly Gln Tyr Lys Leu Gly Ser Lys Thr Gly
                   110                 115                 120
   Pro Gly Gln Lys Ala Ile Leu Phe Leu Pro Met Ser Ala Lys Ser
                   125                 130                 135
<210> 5
   <211> 867
   <212> DNA
   <213> Homo sapiens
<400> 5
   ctggtgggtg tggggggtgg agatgtagaa gatgtgacgc cgcggcccgg 50
   cgggtgccag attagcggac gcggtgcccg cggttgcaac gggatcccgg 100
   gcgctgcagc ttgggaggcg gctctcccca ggcggcgtcc gcggagacac 150
   ccatccgtga accccaggtc ccgggccgcc ggctcgccgc gcaccagggg 200
   ccggcggaca gaagagcggc cgagcggctc gaggctgggg gaccgcgggc 250
   gcggccgcgc gctgccgggc gggaggctgg ggggccgggg ccggggccgt 300
   gccccggagc gggtcggagg ccggggccgg ggccggggga cggcggctcc 350
   ccgcgcggct ccagcggctc ggggatcccg gccgggcccc gcagggacca 400
   tggcagccgg gagcatcacc acgctgcccg ccttgcccga ggatggcggc 450
   agcggcgcct tcccgcccgg ccacttcaag gaccccaagc ggctgtactg 500
   caaaaacggg ggcttcttcc tgcgcatcca ccccgacggc cgagttgacg 550
   gggtccggga gaagagcgac cctcacatca agctacaact tcaagcagaa 600
   gagagaggag ttgtgtctat caaaggagtg tgtgctaacc gttacctggc 650
   tatgaaggaa gatggaagat tactggcttc taaatgtgtt acggatgagt 700
   gtttcttttt tgaacgattg gaatctaata actacaatac ttaccggtca 750
   aggaaataca ccagttggta tgtggcactg aaacgaactg ggcagtataa 800
   acttggatcc aaaacaggac ctgggcagaa agctatactt tttcttccaa 850
   tgtctgctaa  gagctga 867